# EUROPEAN PATENT SPECIFICATION

(11) **EP 3 871 222 B1**
(45) Date of publication and mention of the grant of the patent: **01.01.2025**
(21) Application number: 19809826.1
(22) Date of filing: 27.11.2019
(51) Int. Cl.: G16B 20/00, G16B 45/00

(54) **VECTOR-BASED HAPLOTYPE IDENTIFICATION**
VEKTORBASIERTE HAPLOTYPIDENTIFIZIERUNG
IDENTIFICATION D'HAPLOTYPES BASÉE SUR VECTEUR

(30) Priority: 27.11.2018 EP 18208717
(43) Date of publication of application: 01.09.2021
(73) Proprietor: KWS SAAT SE & Co. KGaA, 37574 Einbeck (DE)
(72) Inventor: WAGNER, Christian, 37574 Einbeck (DE); NEMRI, Adnane, 37079 Göttingen (DE); REINHARDT, Franz-Josef, 37574 Einbeck (DE)
(74) Representative: Richardt Patentanwälte PartG mbB
(86) International application number: PCT/EP2019/082673
(87) International publication number: WO 2020/109356

(56) References cited:
- US-A1- 2004 023 275
- US-A1- 2004 241 730

## Description

### Field of the invention

The invention relates to the field of bioinformatics, and more particularly to a computer implemented method for identifying haplotypes.

### Background and related art

The identification of the haplotype of an organism (also known as "haplotype phasing") refers to the process of estimation of haplotypes from genotype data. Genomic sequence information is collected at a set of polymorphic sites from a group of individuals or from different tissue samples of the same individual. Then, statistical algorithms are applied on the genomic information for estimating haplotypes. Haplotype determination may allow identifying and characterizing the relationship between genetic variation and for example disease susceptibility.

Some haplotype phasing approaches use a multinomial model in which each possible haplotype consistent with the sample is given an unknown frequency parameter and these parameters were estimated with an expectation-maximization (EM) algorithm. Most of these approaches are only able to handle small numbers of genomic features at once. For larger numbers of markers, those algorithms are computationally expensive and lose accuracy by using suboptimal models for haplotype frequencies. Other approaches utilize some form of hidden Markov model (HMM) to carry out inference of the joint distribution of haplotypes. For example, the PHASE algorithm was used to estimate the haplotypes from the HapMap Project. However, PHASE was limited by its speed and was not applicable to datasets from genome-wide association studies (GWASs).

The fastPHASE and BEAGLE methods introduced haplotype cluster models applicable to GWAS-sized datasets. The BEAGLE method, for example, is implemented in the Beagle Software from Brain Browning (University of Washington, Seattle). The Beagle's phasing algorithm is described in S R Browning and B L Browning (2007) "Rapid and accurate haplotype phasing and missing data inference for whole genome association studies by use of localized haplotype clustering". Am J Hum Genet 81:1084-1097 doi:10.1086/521987. The Beagle's genotype imputation algorithm is described in B L Browning and S R Browning (2016): "Genotype imputation with millions of reference samples", Am J Hum Genet 98:116-126, doi:10.1016/j.ajhg.2015.11.020. The Beagle's genotype imputation algorithm is described in B L Browning and S R Browning (2013): "Improving the accuracy and efficiency of identity-by-descent detection in population data. Genetics 194(2):459-71, oi:10.1534/genetics.113.150029".

US 2004/023275 A1 discloses a method for analyzing a matrix constructed from genetic positions and different sources, wherein sequentially each SNP haplotype sequence from a source is com- 5 pared to a data of the SNP haplotype patterns from a pattern set, wherein if the SNP haplotype se- quence being compared is not consistent with any of the SNP haplotype patterns, then the SNP hap- lotype sequence is added to the pattern set and retained in the data set. If the SNP haplotype se- quence is consistent with more than one SNP haplotype pattern, then the SNP haplotype sequence is not added to the pattern set and is retained in the data set. If the SNP haplotype sequence is con- 10 sistent with one SNP haplotype pattern, ambiguities in the consistent SNP haplotype pattern are identified. Ambiguities are resolved using non-ambiguous information from a first SNP haplotype pattern sequence, ultimately building a final SNP haplotype block set of non-overlapping SNP haplotype blocks that include all SNP positions in a set of overlapping SNP haplotype blocks. US 2004/023275 A1 does not suggest to calculate a vector for each cell in the matrix.

Many haplotype phasing approaches are computationally highly demanding, are too slow or too inaccurate to be used in many use case scenarios. Some approaches are too slow to process whole-genome sequences, or can only process specific types of genomic variances, e.g. SNPs. Other approaches, in particular statistical methods, require large data sets comprising a large number of individuals in order to provide statistically significant results. Some approaches are affected by two or more of the above-mentioned problems.

### Summary

It is an objective of the present invention to provide an improved computer-implemented method for identifying haplotypes, and a corresponding storage medium and computer system. The present invention also provides methods for creating a genetically modified organism that comprises a new nucleotide sequence encoding a desired trait, a method of identifying one or more genetic markers, a method of identifying a germplasm whose genome is associated with a desired gene, trait or phenotype, a method of screening on a germplasm, a genetic marker indicative of the presence of a particular gene, trait or phenotype, a method of using the marker for selecting a germplasm and a chip comprising the marker as specified in the independent claims. Embodiments of the invention are given in the dependent claims. Embodiments of the present invention can be freely combined with each other if they are not mutually exclusive.

In one aspect, the invention relates to a computer-implemented method for identifying haplotypes in a set of sources of genetic information. The set of sources of genetic information is a population of organisms or a set of tissues of one or more organisms. The method comprises:
- providing a 2D matrix. The 2D matrix comprises a first and a second dimension and a plurality of 2D matrix cells. The first dimension represents a sequence of genomic positions. The second dimension represents an ordered list of the sources of genetic information. Each of the plurality of cells has assigned via its respective location in the 2D matrix one of the genomic positions and one of the sources of genetic information. Each of the plurality of cells comprises a genomic feature that was observed in the cell's assigned source of genetic information at the cell's assigned genomic position.
- computing, for each of the cells, a vector. The vector comprises multiple elements respectively representing one source in the set of sources of genetic information. Each of the elements of the vector comprises an identity indicator. The identity indicator is a data value indicative of whether the genomic feature comprised in the cell is identical to a genomic feature observed in the source of genetic information represented by said vector element at the genomic position assigned to the cell.
- comparing the vectors with each other for identifying two or more continuous or discontinuous blocks of cells in the 2D matrix that have similar vectors, wherein the vector similarity is computed as a function of the number of different alleles shared by the two compared vectors; and
- outputting the identified blocks of cells, each identified block of cells representing a haplotype observed in the set of sources of genetic information.

The above-mentioned features may be advantageous, because the vector-based determination of similar 2D matrix cells (cells having similar vectors) may be computationally cheap and may be parallelizable and hence scalable. This benefit will become even more important in the future as the available sequence information of many species, e.g. crop species, will increase by the introduction of new sequence technologies. The vector-based determination of the haplotypes may allow rapidly performing whole-genome analysis for a large data set comprising the whole-genome sequences of hundreds or even thousands of individuals or tissue samples. The vector creation for each data point (2D matric cell representing a particular genomic position and a particular organism or tissue) can be processed in parallel. Also, the distance calculation can be performed in parallel. Therefore, the proposed method is suitable for large scale calculations. In contrast to that, the current implementation of some linkage-disequilibrium-based haplotyping methods cannot process maker data whose size exceeds 600 Kbyte. The above-mentioned aspect is particularly beneficial, as many existing algorithms cannot cope with the ever-increasing amount of sequencing information that has been made available by the advancement in rapid sequencing technologies. With recent technological advances, enormous amounts of genotype data are being generated, e.g. from genome-wide SNP microarrays and from ever more affordable whole-genome and whole-exome sequencing tools. Because sequence and SNP array data generally take the form of unphased genotypes, one does not directly observe which of the parental chromosomes a particular allele falls on. This information, however, may quickly be derivable based on the computer-implemented haplotype identification described herein for embodiments of the invention.

In a further beneficial aspect, vector-based computation may not require large datasets comprising a large number of organisms or tissue samples. It may not require the construction of complex models and may not require the application of complex statistical algorithms. In contrast to statistics- and linkage-disequilibrium-based haplotyping, the vector-based haplotype identification method may operate on populations with smaller numbers of individuals, e.g. with sets of less than 11, or even less than 5 organisms or tissue samples acting as sequence information sources. For example, the set of sources of genetic information can have 2 to 10, or only 2 to 5 elements.

In a further beneficial aspect, the vector-based haplotype identification method may operate with a very broad variety of different types of genomic features and associated genetic variations that that can be used, for example, in genome-phenotype association studies. For example, the vector-based haplotype identification method may process a variety of different genomic features, whereby a genomic feature can be, for example, an individual nucleotide, an insertion/deletion variation (INDEL) of one or more nucleotides, a gene- or exon presence or absence variation (PAV), the presence or absence of a simple sequence repeat marker (SSR), an identifier of a nucleotide-sub-sequence of predefined length, an identifier of a unique nucleotide-sub-sequence observed in a multiple-sequence-alignment (MSA) of the genomes of the sources of genetic information, an amplified fragment length polymorphism (AFLP), or a combination of two or more of the above-mentioned feature types. Hence, the vector-based haplotype identification method can universally be applied without major algorithmic adaptations in a variety of use case scenarios.

In a further beneficial aspect, the vector-based haplotype identification method may be able to consider multiallelic genomic features, e.g. quantitative trait loci (QTLs), and even MSAs. Hence, the method may be applicable for various different marker types (see above). This aspect may also increase the accuracy, because if a region of the genome should show a low variability and information richness in respect to one particular genomic feature type, e.g. INDELS, the same genomic region may show sufficient variability in respect to another feature type, e.g. SNIPs, to allow for a fine-grained identification of genomic variances and for a high-resolution identification of associations of genomic feature variations on the one hand with genes, traits or phenotypes on the other hand. This may increase the accuracy of the haplotype identification as well as the subsequent identification of predictive markers and/or the subsequent identification of organisms or germplasms suitable for use in a breeding project.

According to an embodiment, the step of providing the 2D matrix is implemented as reading the 2D matrix from a volatile or non-volatile storage medium. The storage medium can be a local storage medium or a remote storage medium that is accessible via a network, e.g. the Internet or an Intranet. The step of providing the 2D matrix can also comprise reading sequence information of each of the sources of genetic information (e.g. from a storage medium and/or from a sequencing machine), instantiating an empty 2D matrix data structure and filling the matrix cells with a genomic feature that was observed in the source of genetic information at the genomic position that correspond to the x and y coordinates of the cell.

The step of outputting the identified blocks of cells can be implemented, for example, by assigning to each of the 2D matrix cells a color, e.g. a background color, that is unique for each unique identified vector. Hence, all cells having the same vector will be assigned the same color. The color-coded 2D matrix (which may or may not comprise a graphical representation of the vectors of the matrix cells) is displayed as a haploblock plot on a display that is operatively coupled to the computer system. In addition, or alternatively, the haploblock plot is printed on paper or sent via a message of any format (e.g. e-mail, SOAP messages, etc.) to another computer system. In addition, or alternatively, the identified haplotypes and/or the haploblock plot are stored on a local or remote non-volatile storage medium.

According to embodiments, the sources of genetic information are genetically unrelated and/or genetically diverse organisms.

According to embodiments, the vector elements of all vectors having the same vector element position represent the same one of the sources of genetic information. For example, in case the set of sources is a population of five organisms O1, O2, O3, O4, O5, each of the computed vector comprises exactly five elements, whereby the first element position (P1) in all vectors represents organism O1, the second element position (P2) in all vectors represents organism O2, the third element position (P3) in all vectors represents organism O3, the fourth element position (P4) in all vectors represents organism O4, the fifth element position (P5) in all vectors represents organism O5. The vector elements (and respective positions) of each of the vectors represent the sources of genetic information in accordance with a predefined order that is the same for all the vectors. In particular, the predefined order can be identical to the order of the list of sources of genetic information represented by the second dimension. For example, the 2D matrix can be graphically represented on a GUI, whereby the names of the sources of genetic information are plotted along the second dimension. The name list can be ordered alphabetically or in accordance with any other order. Preferably. The element position of the vectors will represent the sources of genetic information in accordance with the order the sources are plotted along the second dimension. This may ease the interpretation of a graphical representation of the vectors, if any, by a human user.

The identity indicator is a data value. According to embodiments, the identity indicator is a binary value. For example, the identity indicator can be one of a pair of two allowed values, e.g. "0 and 1" or "TRUE and FALSE" or "ABSENT and PRESENT" or "IDENTICAL and DIFFERENT".

According to embodiments, the identification of the two or more continuous or discontinuous blocks of cells in the 2D matrix that have similar vector comprises computing the Euclidian distance between any two of the computed vectors and determining all cells whose vectors have an Euclidian distance below a predefined distance threshold value to be member of a continuous or discontinuous block of cells having similar vectors.

According to other embodiments, the distance between any two of the computed vectors is computed as a derivative of the Euclidian distance. For example, the final difference score of two vectors could be computed by computing, in a first step, the Euclidian distance of the two vectors, wherein the Euclidian distance value positively correlates with the number of elements in the two compared vectors which correspond to the same vector position but comprise different identity indicators ("mismatch elements"). Then, the Euclidian distance score is modified in a second step, e.g. by increasing the distance score in case the number of mismatch elements exceeds a predefined threshold.

According to alternative embodiments, the distance between any two of the computed vectors is computed as a derivative of the number of different alleles that are covered and shared by the two compared vectors. The higher the number of shared alleles (which do not correspond to duplicates of a single, particular allele), the higher the similarity score of two compared vectors. For example, the number of shared alleles can be computed as an alternative to or in addition to the Euclidian distance that may be computed on the level of single nucleotides. The computation of an allele frequency based similarity score comprises identifying alleles in the genome sequences of the two compared sources of genetic information, identifying duplicates of particular alleles, and determining the number and types of alleles covered and represented by a vector. The vector similarity is computed as a function of the number of different alleles shared by the two compared vectors. If the two compared vectors share multiple copies of the same allele, this does not increase the similarity score or does at least not increase the similarity score linearly. According to some embodiments, sharing multiple duplicate alleles may even decrease the similarity score.

The above described approaches for computing the allele frequency and the number of shared alleles for determining the similarity of two vectors may be particularly advantageous for computing the similarity of vectors which completely or partially represent repetitive genome regions. A vector similarity that is computed as a derivative of the number of shared unique (non-duplicate) alleles may further have the advantage that the computed similarity score may be used as a kind of quality score. Like linkage-disequilibrium-based approaches, the allele-frequency-based similarity score computation may allow determining vectors, vector-similarity scores and/or genomic markers of lower quality which due to their repetitiveness do not allow to draw conclusion on heredity.

According to other embodiments, the identification of the two or more continuous or discontinuous blocks of cells comprises identifying two or more continuous or discontinuous blocks of cells in the 2D matrix that have identical vectors and selectively using these identified blocks of cells as the block of cells having similar vectors.

Evaluating the identity of vectors may be beneficial, because identity of data values can be determined highly efficiently. For example, the identity can be determined based on a bitwise comparison of identity indicators stored in the elements of the respective vectors. A complex computation of distance/similarity values and a numerical comparison of the obtained distance value with a threshold is not necessary. This may increase the scalability and performance of the method.

According to embodiments, the vectors are computed in parallel by at least two different processing units. In addition, or alternatively, the vectors are compared with each other in parallel by at least two different processing units.

According to some embodiments, the two or more different processing units are two or more central processing units (CPUs).

According to alternative embodiments, the vector generation and/or vector comparison is performed on two or more Graphics Processing Units (GPUs) in parallel. GPUs typically handle computation only for computer graphics. While GPUs operate at lower frequencies than most CPUs, they typically have many times the number of cores. Thus, GPUs can process far more pictures and graphical data per second than a traditional CPU. Using GPUs for parallel computation may be beneficial as current standard computers often come with one or more video cards or graphics chips which comprise a plurality of GPUs. So, performing the haplotype identification on multiple GPUs may allow massive parallelization even on a standard computer that comprises only a single or a small number of standard CPUs. By using GPUs, even a single CPU framework allows parallel execution of the vector-based haplotype identification method.

According to some embodiments, the genomic features are of a feature type selected from a group comprising:
- an individual nucleotide;
- an insertion/deletion variation (INDEL) of one or more nucleotides;
- a gene- or exon presence or absence variation (PAV);
- a presence or absence of a simple sequence repeat marker (SSR);
- an identifier of a nucleotide-sub-sequence of predefined length;
- an identifier of a nucleotide-sub-sequence observed in a multiple-sequence-alignment (MSA) of the genomes of the sources of genetic information;
- a combination of two or more of the above-mentioned feature types.

According to some embodiments, the sequence of genomic positions represented by the first dimension covers two or more different chromosomes.

This may be beneficial, because in contrast to statistics- and linkage-disequilibrium-based haplotype phasing methods, the vector-based haplotype identification method can generate a genome-wide set of vectors. This makes it possible to trace linked inherited markers across multiple different chromosomes.

According to embodiments, the source of genetic information is a haploid organism or a tissue of an haploid organism or a tissue whose cells are in haploid chromosomal state.

According to other embodiments, the source of genetic information is a diploid organism or a tissue of a diploid organism or a tissue whose cells are in diploid chromosomal state.

According to other embodiments, the source of genetic information is a polyploid organism or a tissue of a polyploid organism or a tissue whose cells are in polyploid chromosomal state, whereby a polyploid cell or organism is a cell or organism having more than two paired (homologous) sets of chromosomes.

According to other embodiments, the two or more different chromosomes covered by the first dimension of the 2D matrix comprise chromosomes contained in the same set of non-homologous chromosomes.

According to other embodiments, the two or more different chromosomes covered by the first dimension of the 2D matrix comprise at least two paired (homologous) chromosomes.

According to other embodiments, the sources of genomic information are diploid (n=2) or polyploid (n>2) organisms or tissue samples of diploid or polyploid organisms, wherein n is the number of complete sets of homologous chromosome. Thereby, the character "n" is a ploidy indicator that corresponds to the number of complete sets of chromosomes in a cell, and hence the number of possible alleles for autosomal and pseudoautosomal genes.

According to some of these embodiments, a 2D matrix and a respective set of vectors is computed for each of the sets of homologous chromosomes. Each vector represents one or more non-homologous chromosomes contained in the same set of homologous chromosomes. Hence, the positions represented by the first dimension of each vector cover one, two or more different (-homologous) chromosomes but does not cover homologous chromosomes. For example, for a population of diploid organisms (2n), two different 2D matrices and respective vector sets can be computed. For a population of tetraploid organisms (4n), four different 2D matrices and respective vector sets can be computed.

According to some embodiments, the information encoded in each of the n vector sets can be aggregated for providing an integrated 2D matrix that is used as a basis for computing an integrated set of vectors and for providing an integrated graphical representation of haplotypes in a single integrated haplo-block plot. If, for example, an adenine (A) is found at position X on chromosome C5_{HC1} of a first set of homologous chromosomes HC1 and a thymine (T) is found at the same position X on chromosome C5_{HC2} of a second set of homologous chromosomes HC2, one could use 'w', which stands for A or T according to the "Handbook on industrial property information and documentation" ST.25 page: 3.25.16 03-25-01 of December 2009, Standard for the presentation of nucleotide and amino acid sequence listings in patent applications". Of course, other nucleotide mismatch encoding schemes could likewise be used. Then, the vector-based comparison, the determination of vector similarity and the identification of haplo-blocks can be performed as described herein for embodiments and examples of the invention, whereby the integrated vectors that were derived from the n different vector sets are used as the basis for identifying haplo-blocks.

According to still other embodiments, the vector computation can be performed such that individual genetic markers (or alleles) are considered as genomic positions. If at a particular genomic position X corresponding to a particular marker K only markers derived from the mother are found, that genomic position X is encoded in the 2D matrix as the genomic feature Kₘₒₜₕₑᵣ or "M". If at said position X only markers derived from the father are found, that genomic position X is encoded in the 2D matrix as the genomic feature K_{father} or "F". If at said position X both markers of the mother and of the father are found, that genomic position X is encoded in the 2D matrix as the genomic feature K_{heterozygote} or "H". It is also possible to encode genomic positions representing gene-wise alleles, not only genetic markers in a 2D matrix in this way. Then, the vector-based comparison, the determination of vector similarity and the identification of haplo-blocks can be performed as described herein for embodiments and examples of the invention based on this 2D matrix.

According to embodiments, the set of sources of genetic information comprises at least three elements.

According to some embodiments, the set of sources of genetic information comprises less than 10 sources, e.g. 2-5 organisms or tissue samples.

As the haplotype determination is performed based on a vector comparison rather than on statistical methods, embodiments of the invention may be applicable and provide accurate results also on small data sets comprising less than 10, and even less than 5 organisms or tissue sample. Statistics-based haplotyping approaches typically cannot deal with such small data sets.

According to some embodiments, the outputting comprises generating a plot. The plot can also be referred to as "haploblock plot". The plot comprises a graphical representation of the 2D matrix, wherein matrix cells comprised in the same identified continuous or discontinuous block of cells have the same color or the same hatching. Different ones of the identified cell blocks have different colors or different hatchings. The cells of the haploblock plot can optionally in some implementation variants comprise a graphical representation of the vector having been computed for the 2D matrix cell. The outputting further comprises displaying the plot on a graphical user interface of a display device, e.g. a screen of the computer system used for computing the vectors and the plot.

The identified and output haplotypes may allow a user of the application program to understand the interplay of genetic variation and phenotypic traits, understanding and interpreting hitherto untyped genetic variation, detecting genotype error, inferring demographic history of human and non-human populations, and inferring points of recombination.

According to embodiments, the computer-implemented method further comprises automatically annotating at least one of the identified blocks of cells with one or more genes located in a genomic region represented by the at least one identified block of cells. In addition, or alternatively, the computer-implemented method further comprises enabling a user, preferably via a GUI, to manually annotate at least one of the identified blocks of cells with the one or more genes.

As each continuous or discontinuous block of cells represents an observed haplotype and corresponds to a respective unique vector, the automated and/or user-based assignment of genes (or other annotated data, e.g. traits or phenotypes) to haploblocks implicitly also involves an assignment of genes (or other annotated data) to the unique vector corresponding to a particular haplotype.

According to embodiments, the computer-implemented method further comprises automatically annotating at least one of the identified blocks of cells with one or more traits observed in the sources of genomic information represented by the at least one identified block of cells. In addition, or alternatively, the computer-implemented method further comprises enabling a user, preferably via a GUI, to manually annotate at least one of the identified blocks of cells with the one or more traits. A trait is an observable property of an organism, a tissue, a cell or a cell component.

According to embodiments, the computer-implemented method further comprises automatically annotating at least one of the identified blocks of cells with one or more phenotypes observed in the sources of genomic information represented by the at least one identified block of cells. In addition, or alternatively, the computer-implemented method further comprises enabling a user, preferably via a GUI, to manually annotate at least one of the identified blocks of cells with the one or more phenotypes. A phenotype is a composition of two or more traits.

Various computer-based systems and applications exist for automatically or semiautomatically annotating genomes with meta data, e.g. genes, traits or phenotypes. For example, Proux-Wéra et al. (2012) in "A pipeline for automated annotation of yeast genome sequences by a conserved-synteny approach", BMC bioinformatics 13. 237. 10.1186/1471-2105-13-237 describe a web-based system that exploits homology and synteny information from other yeast species stored in the Yeast Gene Order Browser (YGOB) database for automatically annotating a new genome sequence with identified introns, tRNA genes and Ty-like elements.

Oellrich A, et al.: "Using association rule mining to determine promising secondary phenotyping hypotheses", Bioinformatics, 2014;30(12):i52-i59 describe an association rule mining approach to the identification of promising secondary phenotype candidates. The predictions rely on a large gene-phenotype annotation set that is used to find occurrence patterns of phenotypes. Applying an association rule mining approach, a plurality of secondary phenotype hypotheses were automatically identified and annotated.

Annotating haplotypes rather than individual genome sequences with genes, traits or phenotypes may be advantageous, because a more coarse-grained association (to haplotypes rather than individual genetic markers) is obtained, that may be processed faster than an association table of annotation data to individual nucleotide positions. This may be particularly advantageous when performing whole-genome association studies for a large number of organisms or tissue samples.

Annotating haplotypes and respective vectors with phenotypic information allows tracking co-inherited phenotypes and traits and allows discovering trait specific genomic regions. Hence, embodiments of the invention provide for a GWAS of vectors/haplotypes and phenotypes or traits.

After the haplotypes (and respective vectors) have been manually and/or automatically annotated with genes, traits or phenotypes, the computer-implemented method optionally further comprises a step of automatically analyzing the identified blocks of cells and their annotated genes for automatically identifying co-inherited genes and associated pathways, or displaying the identified cell blocks in association with their annotated genes via a GUI for enabling a user identifying co-inherited genes and associated pathways.

Embodiments of the invention may be beneficial because they provide a computer-implemented haplotype identification method that may allow tracing the co-inheritance of genomic features and associated other features over several generations and for many organisms quickly and reliably. In particular when the identified haplotypes are annotated with additional information such as genes, traits or phenotypes, the information contained in the identified and annotated haplotypes may be of great value for many application scenarios. For example, in the context of a breeding project, the option to identify genes, traits or phenotypes which are all associated with a particular haplotype is highly beneficial as it may pinpoint associations between (easily detectable) genomic features (such as SNPs) with genes, traits or phenotypes. Hence, it may not be necessary to wait until a germplasm has reached the necessary age to develop a certain phenotypic trait like the size, shape or color of the fruit. It may be more efficient to use quick genetic tests, e.g. a DNA chip, to determine whether or not the germplasm comprises markers being indicative of a haplotype known to be associated with one or more desired traits or phenotypes. In a further beneficial aspect, the vector-based haplotyping may allow quickly identifying haplotypes in many different generations, thereby tracking blocks of coupled inheritance (haploblocks) within a population over many generations.

In a further aspect, the invention relates to performing an association study of the identified haplotypes with their respectively annotated genes, traits and/or phenotypes. This may allow or facilitate trait- or phenotype specific target gene discovery by identification of probable metabolic or signaling pathway connections. Performing association studies on the haplotype level may increase performance in comparison to performing these studies on the level of individual genetic markers.

The association studies can in particular be GWASs. That compare the haplotypes of a population of organisms having varying genotypes for a particular trait or phenotype. The population may comprise organisms afflicted with/showing a particular phenotype or trait and may comprise other organisms without this phenotype or trait ("controls"). This approach is known as phenotype-first, in which the individuals are classified first by their phenotypes or trait(s) (as opposed to an alternative but likewise suitable "genotype-first" approach). Each individual gives a sample of DNA, from which millions of genetic variants are read using a DNA chip, e.g. a SNP array. Preferably, the chip comprises DNA probes adapted to selectively bind genetic markers which have been identified as described herein for embodiments of the invention. In particular, the chip may comprise, for each of the identified haplotypes in a training population, a predefined minimum set of genetic markers which are unique for the respective haplotype. If one type of the genomic feature (e.g. a SNP) or haplotype is more frequent in individuals with the phenotype or trait, the genomic feature or haplotype is said to be associated with the phenotype or trait. The associated genomic features or the haplotype are then considered to mark a region of the individual's genome that may influence the probability that the phenotype or trait risk is observed in an individual, e.g. may indicate the risk of an individual of a particular species to have a disease. In this case, this genomic feature or a particular haplotype is also referred to as "marker" of this phenotype or trait. GWA studies investigate the entire genome, in contrast to methods that specifically test a small number of pre-specified genetic regions. Hence, GWAS is a non-candidate-driven approach, in contrast to gene-specific candidate-driven studies.

According to embodiments of the invention, a GWA is applied on the genomes of all organisms of a population in order to identify genomic features (e.g. SNPs and other comparatively small-scale variants in DNA) or haplotypes associated with a phenotype or trait. Although a GWA cannot reveal causal relationships, the results of a GWA can form the basis for further investigations that may reveal causal effects. Performing a GWA on the haplotype level rather than on the level of individual genomic features may significantly improve the scalability and speed of the method.

According to embodiments, the met further comprises identifying, for each of the identified haplotypes, a predefined minimum number of genetic markers being selectively indicative of the presence of said haplotype. The predefined minimum number is independent of the length of the genomic sequence covered by the haplotype. Then, selectively the identified markers are used for performing an association study in a plurality of further sources of genetic information (e.g. in a different population of organisms or in a different set of tissue samples). The association study determines the co-occurrence of the identified genetic markers in the genomes of the other sources on the one hand and of genes, traits or phenotypes observed in the other sources on the other hand.

This may be beneficial, because the haplotype-based identification of genetic markers which are particular for a haplotype may allow performing (genome wide) association studies based on a selection of genetic markers that is more coarse-grained and hence computationally less demanding than approaches that simply use one marker for each defined sub-sequence of e.g. about 100.000 nt. On the one hand, the haplotype-based marker identification improves precision of marker based GWAS as linkage drag effects are avoided or at least reduced. This may improve predictability of genomic selection approaches, because the presence of haploblocks and their respectively associated genes, traits or phenotypes are considered instead of single marker positions.

Applicant has observed that the use of equidistant genetic markers may reduce the accuracy of genomic association studies and the quality of selecting the appropriate genotypes in breeding projects. This is because some genomic regions show a large allelic variability and comprise a plurality of suitable marker sequences while other genomic regions don't. Regions with high marker density (many markers) are often overvalued in genomic association studies, even if these markers are irrelevant for the respective trait to be examined. For example, a plurality of the approximately equidistant genetic markers may actually not provide any additional useful information and rather make the dataset more redundant and even "biased" as these genetic markers may relate to and be associated with the same phenotype or trait. Embodiments of the invention avoid these downsides by simply determining a predefined number of markers per identified haplotype irrespective of the length of the genomic sequence covered by this haplotype. Thereby, co-inherited genomic sub-sequences are considered only once irrespective of the length of the genomic sequence covered by the haplotype. Hence, determining a predefined minimum number of genetic markers per identified haplotype within the genomic sequence covered by said haplotype may increase accuracy of GWASs and of any biological project based on the data provided by these association studies, because co-inherited sub-sequences are basically represented by the same or a similar number of genetic markers. Correspondingly, the genotyping of organisms and tissues based on this specific marker set is more robust against length variations of co-inherited sub-sequences and the resulting variability of the numbers of genetic markers that can be detected in this subsequence. In particular the accuracy of selecting the right genome/germplasm for breeding based on haplotype-specific genetic markers has been observed to be higher than the accuracy of state-of-the-art methods using haplotype-independent marker sets for genotyping.

In a further beneficial aspect, performing the genotyping selectively on the above-mentioned haplotype-specific genetic markers may allow reducing the complexity and computational workload associated with genotyping organisms using conventional, genotyping DNA chips whose probes cover a large number of markers derived from many different sources and plant genera. For example, the MaizeSNP50 DNA Analysis Kit of Illumina is a DNA chip that enables the interrogation of genetic variation across over 30 diverse maize lines. The SNP content of the chip is selected from several public and private sources and contains probes for more than 50,000 validated markers derived from the B73 reference sequence. The chip presents an average of greater than 25 marker-specific probes per mega base (Mb), providing ample SNP density for robust whole-genome genotyping studies. According to embodiments, only a subset of those marker-specific probes (i.e., probes for the above-mentioned haplotype-specific markers) is used for genotyping a Maize germplasm. Applicant has observed that the accuracy of determining the genomic-selection-correlation (trait prediction vs. trait performance) could be significantly increased by selectively using probes for markers identified on a per-haplotype basis. For example, the accuracy could be increased from 0.6 to 0.7 for Maize in respect to a particular trait.

According to embodiments, genome-wide association studies are performed based on vectors or haplotypes (rather than individual genetic markers) which have been annotated with phenotypes or traits for identifying any one of the following association, whereby each association represents an observed co-occurrence of two entities with a co-occurrence frequency that is higher than the expected co-occurrence frequency given the occurrence frequencies of the respective individual entity: vector-gene associations, vector-trait-associations, vector-phenotype-associations. The associations can be identified, for example, using statistical approaches known from conventional genome-wide association studies.

Haplotype-based association studies may have the advantage that a plurality of genomic sequences and genetic markers can be integrated into a single haploblock independent from their physical distance. This can help to discover epistatic genetic linkages for instance. The 'epistatic genetic linkage' is illustrated according to embodiments of the invention via the continuous or discontinuous set of matrix cells identified to have the same vector and to represent the same haploblock, whereby the haploblock may cover genomic locations in several chromosomes. For example: If one always observes the same haploblock comprising specific genomic regions in chromosomes 1, 3 and 7 in plants which exhibit a certain characteristic (trait) such as drought tolerance, one can conclude that this discontinuous haploblock is necessary for the manifestation of this trait and that an epistatic genetic linkage exists.

In a further aspect, the invention relates to a method of creating a genetically modified organism that comprises a new nucleotide sequence encoding a desired trait. The method comprises:
- performing the computer-implemented method for identifying haplotypes according to any one of the embodiments and examples described herein, whereby the set of sources of genetic information are a population of organisms. Each of the organisms comprises or consists of a germplasm. The method is performed for identifying consecutive or non-consecutive cell blocks representing haplotypes of this population;
- identifying one of the identified haplotypes of the population that is associated with (e.g. statistically significantly correlates with) a further desired trait;
- genetically modifying the least one organism by integrating the new nucleotide sequence selectively within the genomic region represented by the one identified haplotype.

For example, if the purpose is to introduce a new gene for drought tolerance into a strain of Zea maise, and if it is known that all or at least some of the individuals of a set of germplasms of this strain already comprise a gene for resistance to a particular pathogen, embodiments of the invention allow rapid and accurate identification of the haplotypes contained in the set of Maize germplasms using the vector-based haplotyping method described above. The haplotypes are then automatically or manually annotated with information concerning phenotypes and traits, including an annotation of a haplotype having been observed to be associated with (have a high frequency of co-occurrence significantly above a statistically expected value) the pathogen resistance. This allows a user to identify at least one haplotype that is associated with an annotation for increased resistance to the pathogen. The user then selects one or more germplasms comprising this at least one identified haplotype with the pathogen resistance annotation and applies a genome editing method (based e.g. on engineered nucleases in particular the CRISPR/Cas9 system) for inserting the drought tolerance gene selectively in a genomic region represented and covered by the at least one identified haplotype. This may ensure that the two desirable traits (pathogen resistance and draught tolerance) will likely be inherited together in the progeny. As the identified haploblocks may cover multiple chromosomes, the haplotype-based selection of gene target regions may provide a greater flexibility of selecting a suitable target region.

In a further aspect, the invention relates to a method of creating a genetically modified organism that comprises a new nucleotide sequence encoding a desired trait. The method comprises:
- performing the computer-implemented method according to any one of the embodiments and examples described herein, whereby the set of sources of genetic information are a population of organisms. Each of the organisms comprises or consists of a germplasm. The method is performed for identifying consecutive or non-consecutive cell blocks representing haplotypes of this population;
- identifying one of the identified haplotypes of the population that is associated with (e.g. correlates with) an undesired trait, the undesired trait being suspected to counteract the desired trait or being suspected to promote introgression of a genomic region suspected to counteract the desired traits;
- genetically modifying the least one organism by integrating the new nucleotide sequence selectively outside of the genomic region represented by the one identified haplotype.

For example, the desired trait may again be draught resistance and the undesired trait may be slow growth of the plant. As the identified haploblocks may cover multiple chromosomes, the haplotype-based selection of gene target regions may provide a greater flexibility of selecting a suitable target region that avoids a situation in which the desirable gene for increased draught resistance is always or typically co-inherited with the undesired trait "slow growth".

In a further aspect, the invention relates to a method of identifying one or more genetic markers respectively associated with a gene, trait or phenotype. The method comprises:
- performing the computer-implemented method of identifying haplotypes and annotating the haplotypes with genes, traits and/or phenotypes as described herein for embodiments of the invention for obtaining haplotypes annotated with genes, traits and/or phenotypes, whereby the set of sources of genetic information is a population of organisms;
- determining, for at least some of the identified haplotypes, one or more candidate genetic markers in the genomic region represented by said haplotype;
- analyzing correlated occurrences of the annotated haplotypes and the determined candidate genetic markers for identifying one or more candidate genetic markers observed to be associated with (frequently co-occur with) one or more genes, traits or phenotypes; and
- using the determined candidate genetic markers as the identified genetic markers.

This may be beneficial, because the association of haploblocks and the identified markers contained therein may allow performing an association study on a fine-grained level, i.e., on the level of the individual markers, and may allow linking the results of this association study to the respective haplotypes comprising the genetic markers.

According to embodiments, the determined candidate genetic markers are sequences at the borders of a haploblock. Preferably, the determined candidate genetic markers are sequences that completely span (cover) a respective one of the identified haploblock(s) or that spat at least the consecutive parts of an identified haploblock. Typically, the candidate genetic markers have a sequence length of about 40-200 nt.

According to some embodiments, the determination of a candidate genetic marker for an identified discontinuous block of cells comprises identifying genomic sequences that span all sub-blocks of the discontinuous block and optionally also the borders of each of said sub-blocks and using the identified sequences as the candidate genetic markers.

According to other embodiments, the determination of a candidate genetic marker for an identified discontinuous block of cells comprises selectively identifying a first genomic sequence that spans the first sub-block of the discontinuous block and optionally also the borders of said first sub-block, selectively identifying a second genomic sequence that spans the last sub-block of the discontinuous block and optionally also the borders of said last sub-block, and using the identified first and second sequences as the candidate genetic markers. Optionally, the candidate genetic marker can in addition span the genomic sequences of one or all of the other sub-blocks which are between the first and the last sub-block of the discontinuous haploblock.

The most appropriate selection of genetic markers depends on the position of the haploblock or haploblocks on the chromo-some and the corresponding genetic context. For example, the presence of highly-repetitive sequences or the presence of highly-condensed genomic sections, for example near the centromere, can influence the selection.

According to embodiments, the determined candidate genetic markers are sequence variants that selectively and uniquely occur in their respective haplotype and not in other identified haplotypes covering the same or other genomic positions. This may be beneficial, because the use of haploblock-specific genomic markers may allow performing an association study on a coarse-grained level, i.e., on the level of individual haplotypes rather than on the level of individual markers, and may allow increasing performance and accuracy in particular for whole genome association studies.

For example, a candidate genetic marker can be SNPs, QTLs, etc.

The genetic markers can be identified using computational approaches. For example, DNA-subsequences corresponding to respective haplotypes can be split into a plurality of short DNA fragments of a defined length, e.g. 4-50 nucleotides ad a plurality of different, shifted "splitting frames". By intersecting the sets of DNA fragments obtained for different haplotypes, and selectively maintaining the DNA fragments which are unique for their respective haplotype, haplotype-specific genetic markers can be identified quickly.

The precision of identifying the borders of the identified haploblocks in the genome of an organism is dependent on the quality and density of the marker determination method and on the granularity of the genomic positions and respective genomic features in the 2D matrix. An ideal case would be complete sequencing of genomes without gaps/lacks of information. This might allow performing a 100% accurate haplotype border determination . In coarse grained genomic features are used for constructing the 2D matrix, e.g. in case of using a 30k SNP chip for obtaining the genomic features, the haplotype border is fuzzy in the range of the distance of the genomic features used.

In a further aspect, the invention relates to a method of identifying a germplasm whose genome is associated with a desired first desired gene, trait or phenotype. The method comprises:
- performing the computer-implemented method for identifying one or more first genetic markers associated with the first desired gene, trait or phenotype in the genomes of individuals of a particular species as described herein for embodiments and examples of the invention. The sources of genetic information are organisms of this species. In general, genetic marker identification can happen on other individuals than those actually used for breeding.
- providing a set of germplasms of this species; and
- identifying one or more first ones of the germplasms whose genome comprises the identified first genetic markers. According to preferred embodiments, in case multiple first germplasms are identified, only the ones of the first germplasms comprising all the identified first genetic markers within a single haploblock or within a minimum number of haploblocks are identified.

This may allow identifying organisms which comprise genetic markers that are associated with desired traits and that preferably have a high chance of being inherited together.

According to an alternative embodiment, one or more first haplotypes (identified e.g. based on the presence of some genetic markers) associated with the first desired gene, trait or phenotype are identified as described herein for embodiments and examples of the invention. Then, one or more first ones of the germplasms whose genome comprises the identified first haplotype are identified.

According to embodiments, the identification of the first germplasms in addition comprises: For each of the provided germplasms:
- providing a genotyping chip; the chip comprises nucleic acid probes respectively adapted to selectively bind to nucleic acid sequences comprising one of the identified first genetic markers;
- applying DNA or RNA obtained from the germplasm on the chip; and
- analyzing the chip carrying the applied DNA or RNA for determining if the genome of said germplasms comprises the identified first genetic markers.

Hence, the vector-based haplotype detection method can be used for identifying haplotypes. Each haplotype can potentially comprise one or more genetic markers which frequently co-occur and are associated with particular genes, traits or phenotypes. In some embodiments, the vector-based haplotype detection method can be used for identifying a subset of the above-mentioned genetic markers which are associated with particular genes, traits or phenotypes and which in addition are uniquely contained in a particular one of the identified haplotypes. This enables more coarse-grained and even faster whole genome association studies.

Instead of a DNA Chip, fluorescence-labeled DNA probes, PCR, Multiplex-PCR etc. could also be used for rapid genotyping of a germplasm or a somatic tissue sample.

According to embodiments, the method of identifying a germplasm whose genome is associated with a desired first gene, trait or phenotype further comprises a step of identifying second ones of the provided germplasms having a genome associated with a desired second gene, trait or phenotype. The method comprises: performing the computer-implemented method genetic marker identification method described herein for embodiments and examples of the invention for identifying one or more second genetic markers associated with the second desired gene, trait or phenotype in the genomes of individuals of the particular species, whereby the sources of genetic information are organisms of this species; and identifying one or more second ones of the germplasms whose genome comprises the identified second genetic markers. According to preferred embodiments, in case multiple second germplasms are identified, only the ones of the second germplasms comprising all the identified second genetic markers within a single haploblock or within a minimum number of haploblocks is identified.

According to embodiments, the method further comprises a subsequent step of selectively propagating the germplasm identified to comprise the identified first genetic markers.

According to other embodiments, the method further comprises a subsequent step of selectively propagating the germplasm identified to comprise the identified second genetic markers.

According to other embodiments, the method further comprises a subsequent step of crossing an individual having a first germplasm comprising the identified first genetic markers with an individual having a second germplasm comprising the identified second genetic markers and selecting progeny carrying the first desired gene, trait or phenotype and carrying the desired second gene, trait or phenotype.

In a further aspect, the invention relates to a method of screening on a germplasm that comprises one or more desired genes, traits or phenotypes. The method comprises: performing the computer-implemented haplotype identification method according to any one of the embodiments described herein, whereby a population of organisms is used as the set of sources of genetic information for identifying consecutive or non-consecutive cell blocks representing haplotypes of the population. The method further comprises identifying an organism in the population whose germplasm comprises one or more desired genes, traits or phenotypes based on the identified consecutive or non-consecutive cell blocks.

For example, selectively those organisms could be identified which comprises a plurality of desired traits or phenotypes within a minimum number of haplotypes, e.g. within a single haplotype. This may allow selectively using those organisms in a breeding project that will likely pass on the desired traits in a way that the traits are co-inherited in subsequent generations.

In a further aspect, the invention relates to a genetic marker being indicative of the presence of a particular gene, trait or phenotype in an organism. The genetic marker is determined by a method comprising:
- identifying consecutive or non-consecutive cell blocks using genomic information of a set of sources of genetic information by performing the computer-implemented method according to any one of the embodiments and examples described herein;
- annotating the identified cell blocks with one or more genes contained in the genomic region represented by one of the blocks and/or
- annotating the identified cell blocks with one or more traits or phenotypes observed in all sources of genetic information represented by the cell blocks; and
- analyzing the annotated blocks of cells for identifying one or more genetic markers being associated with the presence of the particular gene, trait or phenotype.

For example, a marker associated with a particular trait is a marker that was observed to co-occur with this trait significantly more often than would be expected based on the frequency of the marker and the trait in the population assuming a random distribution of the marker and the trait. Hence, a marker associated with a trait can be considered to be a marker being indicative of said trait.

In a further aspect, the invention relates to the use of the genetic marker according to any one of the embodiments described herein for selecting germplasm that comprises one or more desired genes, traits or phenotypes.

In a further aspect, the invention relates to a chip comprising one or more nucleic acid probes adapted to selectively bind to nuclei acid molecules comprising one or more genetic markers according to any one of the embodiments described herein.

In a further aspect, the invention relates to a method for selecting individuals of a population of organisms in a breeding program. The method comprises the steps of:
- growing a genetically diverse population of training organisms;
- phenotyping the genetically diverse population of training organisms to generate a phenotype training data set, the phenotype training data set being indicative of phenotypes and traits of the training organisms;
- obtaining a genotype training data set comprising genetic information across the genome of each of the training organisms, wherein the genetic information comprises a plurality of genetic markers identified in the genome of the training organism in accordance with a computer implemented method that identifies genetic markers within haplotypes; in particular, the genetic markers identified can comprise or consist of genetic markers that are unique for and are indicative of the haplotype DNA sequence within which they were identified;
- obtaining an association training data set by associating the phenotype training data set with the genotype training data set, the association training data set being indicative of associations of some of the genetic markers and some of the phenotypes or traits; the association typically indicates a higher-than-random co-occurrence of a genetic marker and a phenotype/trait;
- genotyping a genetically diverse population of breeding organisms using the plurality of genetic markers; and
- selecting breeding organisms from the genotyped, genetically diverse population of breeding organisms using the association training data set to select breeding pairs likely to generate progeny with one or more desired genes, traits or phenotypes.

The selection is based on the genetic markers identified in the genotyping step and based on the association training data set that indicates the phenotypes or traits respectively associated with one of the genetic markers. For example, the genetic marker-based association data set can be obtained as described in WO2016/069078 A1.

Alternatively, a method for selecting individuals of a population of organisms in a breeding program is provided that uses association data obtained on the level of haplotypes rather than individual genetic markers. The method comprises:
- growing a genetically diverse population of training organisms;
- phenotyping the genetically diverse population of training organisms to generate a phenotype training data set, the phenotype training data set being indicative of phenotypes and traits of the training organisms;
- identifying consecutive or non-consecutive cell blocks representing training haplotypes, the training haplotypes being haplotypes of the training organisms, by performing the computer-implemented method according to any one of claims 1-9, thereby using the genetically diverse population of training organisms as the set of sources of genetic information;
- obtaining an association training data set by associating the phenotype training data set with the training haplotypes, the association training data set being indicative of associations of some of the training haplotypes and some of the phenotypes or traits;
- identifying consecutive or non-consecutive cell blocks representing breeding haplotypes of a genetically diverse population of breeding organisms, the breeding haplotypes being haplotypes of the breeding organisms, by performing the computer-implemented method according to any one of claims 1-9, thereby using the genetically diverse population of breeding organisms as the set of sources of genetic information;
- applying the association training data set on the identified breeding haplotypes for selecting breeding pairs likely to generate progeny with one or more desired genes, traits or phenotypes.

In contrast to the genetic-marker based generation of the association data, the association training data generated by associating the phenotype training data set with the haplotypes may be more scalable. This is because the number of haplotypes identified in an organism is typically much smaller than the number of genetic markers. Each haplotype can be represented by a few numbers or even a single haplotype-specific genetic marker or even by a single haplotype identifier like "H2389" that abstracts away from a particular DNA sequence. This reduces the amount of data that has to be loaded, processed and stored. The use of haplotype-based association data for selecting suitable breeding organisms may also be more accurate, because the number of probes used in state-of-the-art genotyping chips for detecting a particular associated trait may vary and from trait to trait and may result in an overestimation of a trait that is covered in a DNA chip by multiple marker-specific probes.

According to embodiments, the method further comprises:
- crossing said selected breeding organisms, and
- optionally, growing the progeny with one or more genes, traits or phenotypes.

According to embodiments, said breeding organisms are inbred or double haploid organisms.

For example, the haplotyping approach could be used once in a maternal pool of genotypes and once in a pool of paternal genotypes, both pools comprising inbred lines and double haploid lines, respectively. From the comparison of identified haplo-blocks one could try to derive the combining ability of genomes in order to identify suitable pairs of parents that produce powerful hybrid offspring (with strong heterosis effect).

According to embodiments the genotypic information for the training individuals further comprises gene expression information, metabolite concentration, or protein concentration.

According to embodiments selection of the breeding organisms from the genetically diverse population of breeding organisms uses in addition a biological model for selecting the breeding pairs.

Various types of biological models that can be used for selecting suitable breeding organisms are known in the art. For example, models with a defined number of traits can be specified and using approximate Bayesian computation (ABC) methods or genomic best linear unbiased prediction (GBLUP) methods.

The models relate genomic features, in particular genetic markers, to traits and phenotypes of interest (e.g. traits and phenotypes affecting the robustness and resistance to diseases, the yield and agronomic performance of an organism). The models comprise explicit or implicit knowledge about the relationships of these genomic features to traits and phenotypes, whereby the knowledge is typically derived from a training set of sources of genetic information and can be used for assessing and predicting the genetic value of organisms of the same species or strain as used in the training set. The models have "learned" associations of genomic markers and phenotypes/traits and are configured to assess, based on genotyping information obtained for a particular organisms or germplasm the phenotypes and/or traits that will be observed in this organism at a later state of development and/or in the offspring of this organism. For example, the genotyping information can comprise the haplotypes identified based on a computer-implemented method according to embodiments of the invention. In addition, or alternatively, the genomic information can comprise genetic markers which are unique for and indicative of a particular haplotype.

A model may comprise additional biological or agricultural knowledge in addition to gene-phenotype relationships. For example, Muchow et al (1990) propose a crop growth model that models corn biomass (BM) growth as a function of temperature and solar radiation as well as of several physiologic traits of the plant. The physiological traits are assigned to one or more respective genetic markers.

In a further aspect, the invention relates to a computer-readable, non-volatile storage medium comprising instructions which, when executed by a processor, cause the processor to perform a computer-implemented method for haplotype identification according to any one of the embodiments and examples described herein.

In a further aspect, the invention relates to a computer system comprising a storage medium and one or more processors. The storage medium comprises a 2D matrix. The 2D matrix comprises a first and a second dimension and a plurality of 2D matrix cells. The first dimension represents a sequence of genomic positions. The second dimension represents an ordered list of sources of genetic information, whereby the sources of genetic information are a population of organisms or a set of tissues of one or more organisms. Each of the plurality of cells have assigned via its respective location in the 2D matrix one of the genomic positions and one of the sources of genetic information. Each of the plurality of cells comprises a genomic feature that was observed in the cell's assigned source of genetic information at the cell's assigned genomic position.

The one or more processors are configured for:
- computing, for each of the cells, a vector. The vector comprises multiple elements respectively representing one of the sources of genetic information. Each of the elements of the vector comprises an identity indicator. The identity indicator is a data value indicative of whether the genomic feature comprised in the cell is identical to a genomic feature observed in the source of genetic information represented by said vector element at the genomic position assigned to the cell;
- comparing the vectors with each other for identifying two or more continuous or discontinuous blocks of cells in the 2D matrix that have similar vectors; and
- outputting the identified blocks of cells, each identified block of cells representing a haplotype observed in the sources of genetic information.

A "2D matrix" as used herein is a computer-interpretable data structure having two dimensions. The data structure can, but does not have to be graphically represented. In some embodiments, the 2D matrix is implemented e.g. as a two-dimensional ARRAY or a two-dimensional VECTOR, whereby ARRAY or VECTOR are data types supported by the programming language used for implementing the haplotype identification program logic. Whether or not the data structure used to provide the 2D Matrix allows dynamic data allocation or data type constraints depends on the program language used and is considered irrelevant in this context. For example, Java supports both the VECTOR and the ARRAY data types, whereby the key difference between Arrays and Vectors in Java is that Vectors are dynamically-allocated. They aren't declared to contain a type of variable; instead, each Vector contains a dynamic list of references to other objects. When a Vector is instantiated, it declares an object array of size initial Capacity. The 2D matrix is not necessarily visually represented, it may simply be a typed or non-typed data structure such as a 2D array or 2D vector. In some embodiments, the 2D matrix is visually represented on a graphical user interface (GUI), e.g. in the form of a matrix of visible cells similar to a spreadsheet canvas. Optionally, each cell of the graphically represented 2D matrix can comprise a visual representation of the vector computed for this cell. In this case, the 2D matrix can be considered as a 3D matrix, whereby the vectors represent the third dimension.

A "vector" as used herein is a computer-interpretable data structure having one dimension. The data structure can, but does not have to be graphically represented. For example, the "vector" can be implemented as Java vector or Java array or as a corresponding data structure in another program language such as C, C++, C# and the like.

A "phenotype" as used herein is a composition of two or more traits of an organism or cell. According to some embodiments, a phenotype is the composite of an organism's observable characteristics or traits.

A "trait" as used herein is an observable property of an organism, a tissue, a cell or a cell component. The "observation" may be performed by any empirically available method. Hence, the observable property can be an optical/visible feature, but can also be a molecular feature, a behavior, a resistance to a pathogen, robustness in respect to an environmental stress factor such as heat or draught, or the like.

A "genomic feature" as used herein is a piece of genomic information that was observed in a particular cell at a particular genomic position. For example, a genomic feature can be the type, absence or presence of a particular nucleotide at a particular single-nucleotide-position. Alternatively, the genomic feature can be an identifier of a particular sub-sequence at a genomic position covering multiple nucleotide positions. The sub-sequence can be a genomic sequence of a predefined length, e.g. 10 nucleotides, or 20 nucleotides, that belongs to a set of unique sub-sequences obtained for a particular genomic region by means of a multiple-sequence-alignment of genomic sequence data obtained from a plurality of sources of genetic information.

A "genomic position" can correspond to one or more nucleotides.

A "quantitative genomic trait locus (QTL)" as used herein is a locus (section of DNA) which correlates with variation of a quantitative trait in the phenotype of a population of organisms. QTLs are identified and mapped by identifying which molecular markers (such as SNPs or AFLPs) correlate with an observed trait. This is often an early step in identifying and sequencing the actual genes that cause the trait variation.

A "amplified fragment length polymorphism (AFLP)" as used herein is data indictive of a presence-absence polymorphism. AFLPs are derived via PCR-based approaches and are used in genetics research, DNA fingerprinting, and in the practice of genetic engineering. Developed in the early 1990s by Keygene, AFLP uses restriction enzymes to digest genomic DNA, followed by ligation of adaptors to the sticky ends of the restriction fragments. A subset of the restriction fragments is then selected to be amplified by using primers complementary to the adaptor sequence, the restriction site sequence and a few nucleotides inside the restriction site fragments. The amplified fragments are separated and visualized on denaturing on agarose gel electrophoresis, either through autoradiography or fluorescence methodologies, or via automated capillary sequencing instruments.

A "genetic marker" as used herein is a gene or DNA sequence with a known location on a chromosome that can be used to identify individuals or species or a particular trait or phenotype that is associated with this marker. The association can be a known co-occurrence frequency that is higher than expected based on a random co-occurrence given the frequency of the genetic marker and the respective phenotype or trait in the population. A genetic marker can be described as a variation (which may arise due to mutation or alteration in the genomic loci) that can be observed. A genetic marker may be a short DNA sequence, such as a sequence surrounding a single base-pair change (single nucleotide polymorphism, SNP), or a long one, like minisatellites.

A "germplasm" as used herein is a living genetic resource such as a seed or tissue that is maintained for the purpose of animal and plant breeding, preservation, and other research uses. These resources may take the form of seed collections stored in seed banks, trees growing in nurseries, animal breeding lines maintained in animal breeding programs or gene banks, etc. Germplasm collections can range from collections of wild species to elite, domesticated breeding lines that have undergone extensive human selection.

A "genome-wide association study (GWA study, or GWAS)", also known as whole genome association study (WGA study, or WGAS), is an observational study of a genome-wide set of genetic variants in different individuals to see if any variant is associated with a trait or phenotype. According to embodiments, GWASs are performed for identifying statistical associations between particular genomic features, e.g. single-nucleotide polymorphisms (SNPs), and traits or phenotypes like resistance to pathogens, growth speed, robustness to environmental stress factors, and the like. In addition, or alternatively, GWASs are performed for identifying statistical associations between particular haplotypes on the one hand and traits or phenotypes on the other hand. Haplotype based association studies may have the benefit of a reduced degree of complexity and a reduced amount of data to be analyzed and hence are particularly suited for WGAS.

The "identification of entity A co-occurring with and/or being associated with the presence of entity B" as used herein means in particular that entity A has been observed to co-occur with entity B more frequently than statistically expected based on the known occurrence frequencies of the respective entities A, B. Various algorithms that can be used for identifying such associations are known from the technical field of "genomic association studies" where various approaches are used for detecting statistically significant associations e.g. between genetic markers and genes, traits and phenotypes.

A "haplotype" as used herein is a collection of genomic features (in particular, specific DNA sequences like specific alleles, SNPs, or the like) that are tightly linked such that they are likely to be inherited together-that is, they are likely to be conserved as a sequence (or "cluster") of genomic features that survives the descent of many generations of reproduction. For example, a set of single-nucleotide polymorphism (SNP) alleles that tend to always occur together (i.e., that are associated statistically) can be identified as a "haplotype". The identification - based on statistical or other means - of tightly linked genomic features that can easily be detected (e.g. SNIPs) and that form a specific haplotype can be used for identifying other such polymorphic sites that are nearby on the chromosome (and which may not correspond to an already known marker). Such information may allow investigating the genetics of phenotypic traits (see the International HapMap Project for identifying haplotypes corresponding to common human diseases). Genotypes measure the unordered combination of alleles at each site, whereas haplotypes are sequences of genomic features, e.g. alleles, that have likely been inherited together from the individual's parents. When there are N heterozygous genotypes present in an individual's set of genotypes, there will be 2^{N} possible pairs of haplotypes that could underlie the genotypes. For example, when N = 2, the following haplotypes exist: AA/TT, AT/TA, TA/AT, and TT/AA. If there are missing genotypes then the number of possible haplotype pairs increases.

A "haploblock" as used herein is the series of continuous or discontinuous blocks of 2D matrix cells sharing the same vector. A haploblock represents a haplotype.

A "molecular marker" as used herein is a molecule that can be used to reveal certain characteristics about the source from which it was taken, e.g. a cell sample, blood sample or tissue sample taken from an organism or germplasm. DNA, for example, is a molecular marker containing information about genetic disorders, genealogy and the evolutionary history of life.

### Brief description of the drawings

In the following embodiments of the invention are explained in greater detail, by way of example only, making reference to the drawings in which:
- Figure 1: is a flowchart of a haplotype identification method;
- Figure 2: is a block diagram of a computer system configured for identifying haplotypes;
- Figure 3: depicts a 2D matrix comprising cells with feature values;
- Figure 4: depicts a 3D matrix comprising vectors in each cell;
- Figure 5: depicts two versions of a haploblock plot;
- Figure 6: is a screenshot of a further haploblock plot;
- Figure 7: illustrates an MSA-based version of a vector-based haplotype identification method; and
- Figure 8: is a block diagram of a DNA chip.

### Detailed description

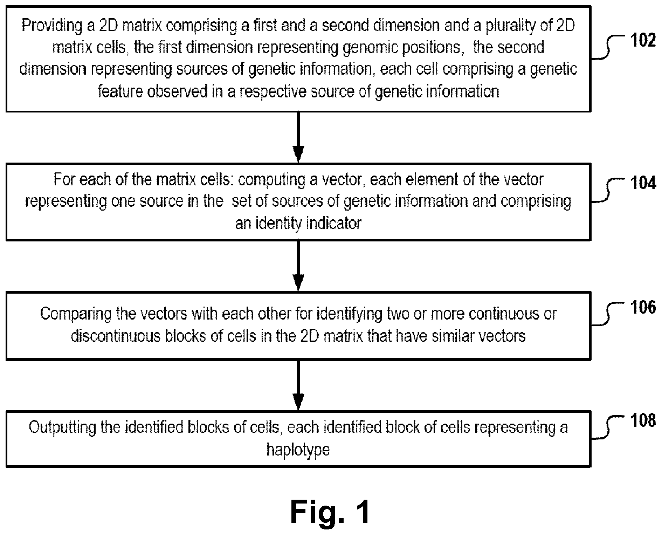

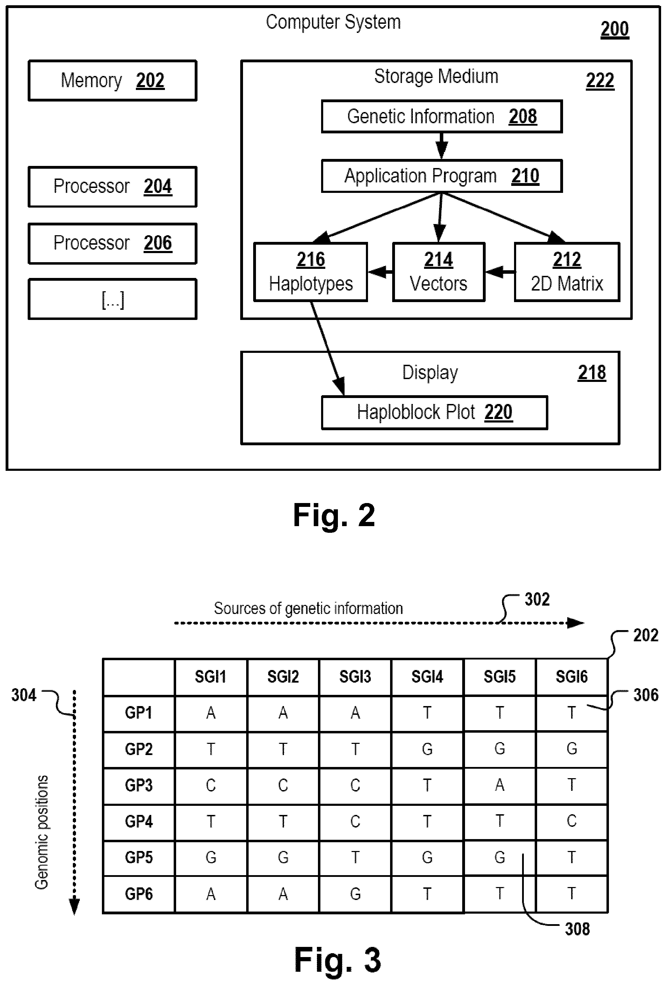

**Figure 1** is a flowchart of a computer-implemented haplotype identification method. In the following, the method depicted in figure 1 will be described by referring also to components of the system depicted in figure 2. The method can be executed, for example, by one or more processors 204, 206 of a computer system 200 executing a haplotype-identification application program 210.

First in step 102, a 2D matrix 202 is provided. For example, the computer system 200 can read, create or otherwise instantiate a data structure, e.g. a vector or an array, that can be used as a container for a two-dimensional matrix of data values. The 2D matrix comprises a first dimension 304 representing a sequence of genomic positions and a second dimension 302 representing an ordered list of sources of genetic information. For example, the sources of genetic information can be a population of organisms. Alternatively, the sources of genetic information can be a set of tissues of one or more organisms of the same or of different species.

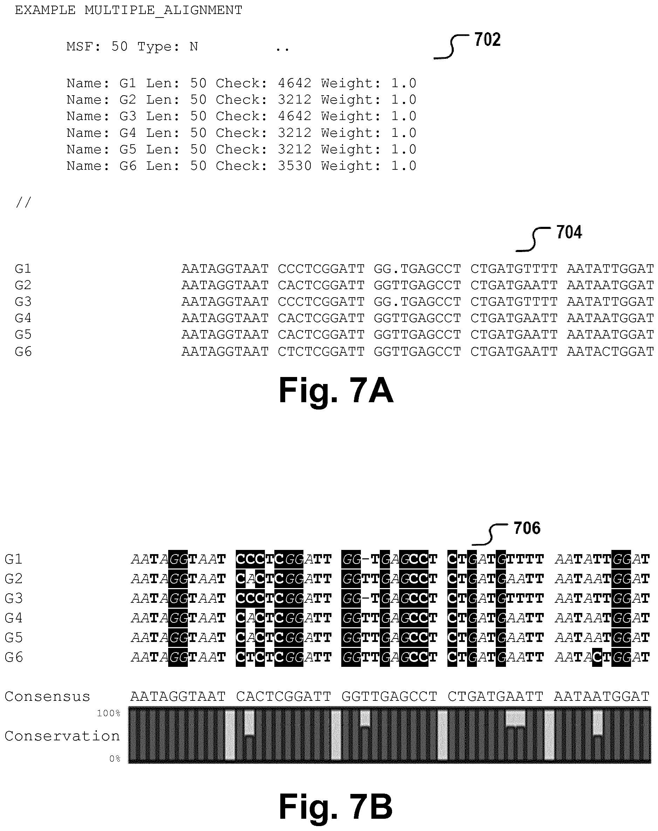

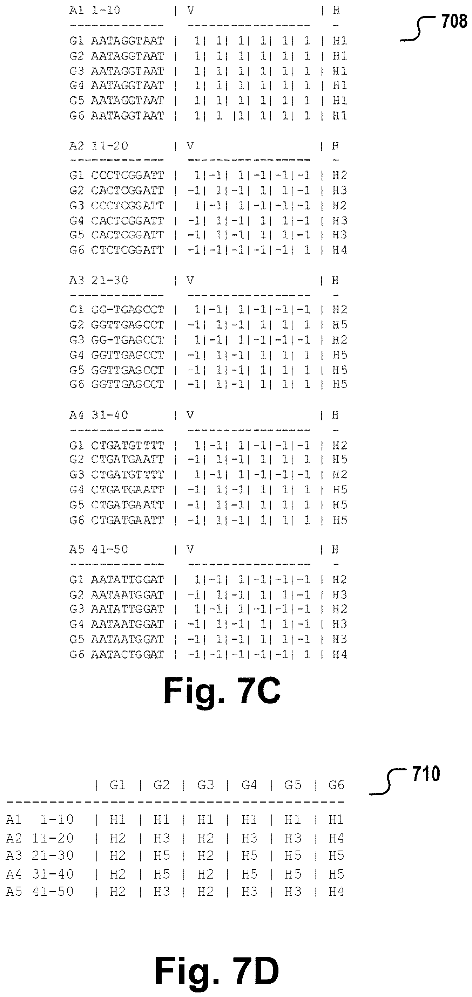

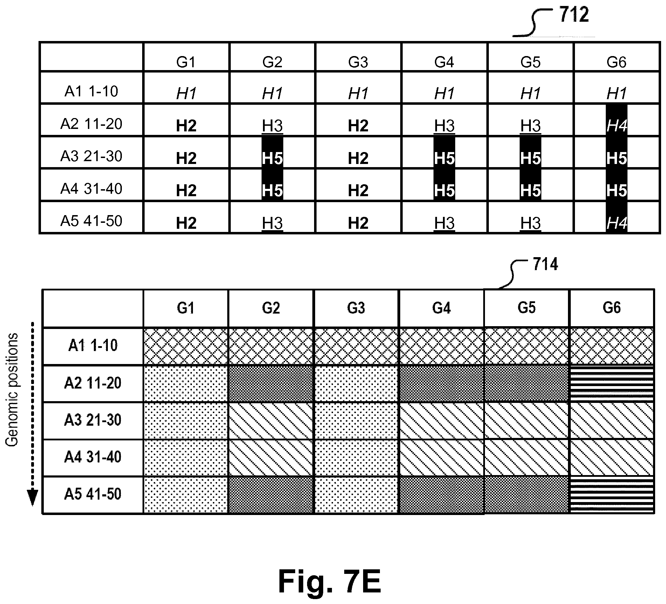

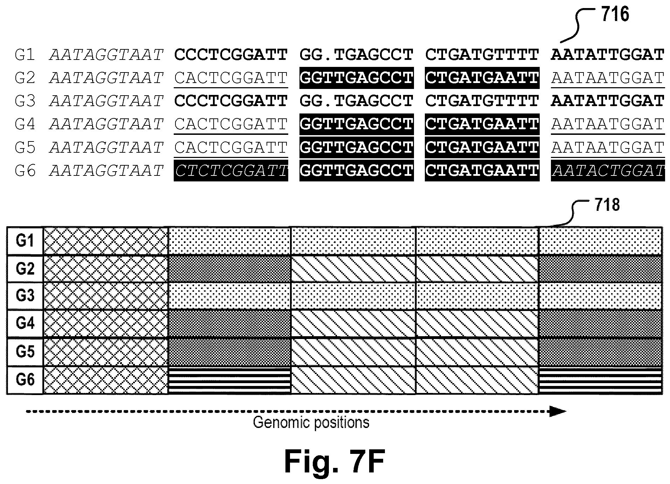

In addition, the 2D matrix comprises a plurality of 2D matrix cells 306, 308. As the cells are matrix cells, each of the plurality of cells has assigned via its respective location in the 2D matrix (in other words, via its x, y coordinates), one of the genomic positions and one of the sources of genetic information. Each of the plurality of cells comprises a genomic feature that was observed in the cell's assigned source of genetic information at the cell's assigned genomic position. For example, if a cell is within a matrix column representing organism "SGI3" and within a row representing genomic position "GP5", the genomic value contained in this call is the genomic feature that was observed in organism "SGI3" at genomic position "GP5". The genomic feature can be, for example, a particular nucleotide. Likewise, in case the genomic position is a sequence of nucleotides of predefined length, e.g. 10 nt, the genomic feature can be an identifier of a unique sub-vector observed in an multi-sequence alignment as described, for example, in figure 7. According to some embodiments, the application program 210 graphically represents and displays the 2D matrix via a graphical user interface (GUI) on an electronic display 218.

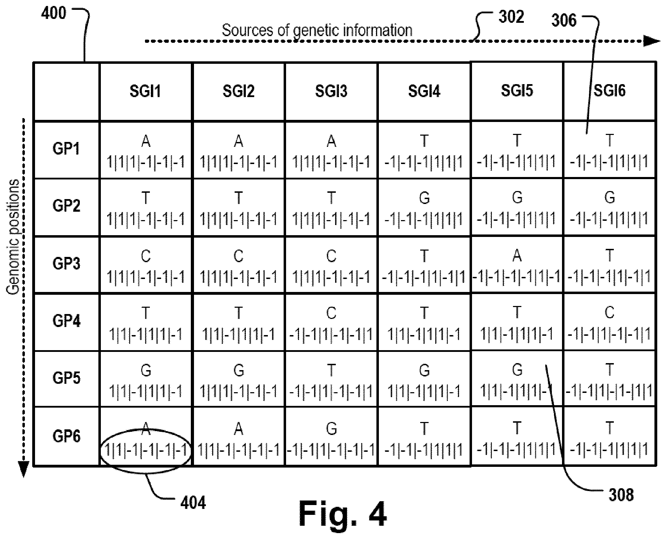

Next in step 104, a vector 404 is computed for each of the cells of the 2D matrix. The vector comprises multiple elements. Each vector element represents a respective one of the sources of genetic information. Hence, in case the second dimension 302 of the 2D matrix covers a particular number S of sources of genetic information, each computed vector comprises S vector elements. Each of the elements of each vector comprises an identity indicator. An identity indicator is a data value indicative of whether the genomic feature comprised in the cell for which the vector was computed is identical to a genomic feature observed at the genomic position assigned to the cell in the one of the sources of genetic information represented by said vector element. This will be explained in greater detail in the description of figures 3 and 4.

According to some embodiments, the graphical representation of the 2D matrix, if any, is supplemented with a graphical representation of the vectors and their identity indicators that were computed for all the matrix cells and are also displayed via the GUI.

The association of the 2D matrix with the vectors computed for each of the matrix cells can be considered as a 3D matrix, whereby the vectors represent the third dimension. As the vector comprises as many vector elements as there are sources of genetic information in the second dimension, the second and the third dimension have the same number of units populated with a data value. As all genomic positions inherited together within a population will get the same vector, each vector could also be referred to as "polymorphism inheritance vector".

Next in step 106, the vectors are compared with each other for identifying two or more continuous or discontinuous blocks of cells in the 2D matrix that have similar vectors. Each identified block of cells represents a haplotype that was observed in the sources of genetic information.

In some embodiments, this step comprises identifying two or more continuous or discontinuous blocks of cells in the 2D matrix that have **identical** vectors. Typically, identity of vectors can be determined faster and with less computational effort than vector similarity/dissimilarity.

Next in step 108, the identified blocks of cells are output. For example, call matrix cells which share the same vector can be highlighted in the same color. The color-codes graphical representation of the 2D matrix can be displayed via a GUI on the electronic display 218 for enabling a user to review the automatically identified haploblocks. As all genomic positions inherited together within a population will get the same vector, those co-inherited genomic positions will be assigned the same color or hatching and will be graphically represented as member of the same haploblock.

**Figure** 2 is a block diagram of a computer system 200 configured for identifying haplotypes in accordance with a computer-implemented method according to embodiments of the invention and as illustrated, for example, in the flow chart depicted in figure 1.

The computer system 200 can be, for example, a standard computer system, e.g. a desktop computer system, a server computer system, or a portable computer system. The portable computer system can be, for example, a notebook, a netbook, a mobile communication device such as a smartphone or a tablet computer. The computer system comprises one or more processors 204, 206. Preferably, the computer comprises a plurality of processors and performs the vector computation and/or vector comparison in parallel on the plurality of processors. The processors can be core processing units (CPUs) or graphical processing units (GPUs). The computer system 200 further comprises or is operatively coupled to a storage medium 222, e.g. a non-volatile storage medium such as a disk storage medium or tape. The storage medium 222 can comprise one or more logical storage volumes and can be based on one or more physical storage units. The physical storage units can be an integral part of the computer system 200 or can be a network storage that is accessible via a network such as the Internet or the Intranet of an organization. The computer system further comprises a main memory 202 where programs and data are kept when the processor(s) is/are actively using them. When programs and data become active, they are copied from the non-volatile storage medium 222 acting as secondary memory into main memory where the processor can interact with them. Preferably, the main memory is a RAM (Random Access Memory).

The storage medium 222 comprises an application program 210 that is configured to use genetic information 208, e.g. whole genome sequences, of a plurality of organisms or tissues for creating a 2D matrix 202, 212. For example, the genetic information 108 can be read from the storage medium 222 or from another remote or local data source. The application program 210 is further configured to compute 104, for each of the matrix cells, a vector 404. Hence, for any 2D matrix 212, the application program 212 computes as many vectors 214 as cells exist in the 2D matrix. In addition, the application program 210 is configured for comparing 106 the vectors 214 with each other for identifying continuous or discontinuous blocks of matrix cells sharing the same vector. These continuous or discontinuous blocks of matrix cells are identified as "haplotypes" 216 and output 108 to a user. For example, the identified continuous or discontinuous blocks of matrix cells can be graphically represented as color-coded matrix cell blocks and displayed to a user via an electronic display 218, e.g. an LCD display of a standard computer system or via a touchscreen of a smartphone.

Thanks to the parallelizability of the vector computation and comparison, thanks to a tremendous reduction of data size and complexity in particular in the context of using sub-vector identifiers as genomic features (see figure 7), the software 210 may be executed even on devices with limited data processing capacities such as smartphones or netbooks.

**Figure 3** depicts a 2D matrix 202 having a first dimension 304 covering six genomic positions GP1-GP6 and a second dimension 302 covering six sources of genetic information SGI1-SGI6, e.g. six different organisms. The matrix comprises cells 306, 308 with genomic feature values in the form of single nucleotide abbreviations: adenine (A), cytosine (C), guanine (G), and thymine (T). The genomic feature "G" in cell 308 indicates that a guanine nucleotide was observed in organism SGI5 at genomic position GP5. The genomic feature "T" in cell 306 indicates that a thymine nucleotide was observed in organism SGI6 at genomic position GP1. Instead of single nucleotides, other forms of genomic features could likewise be used to fill the cells, e.g. SNPs, identifiers of unique sub-vectors obtained in a MSA, INDELs, and others. However, for the sake of simplicity, only the "observed single nucleotide" type of genomic feature is depicted and described in figures 3 to 6.

**Figure 4** depicts a 3D matrix 400 comprising one vector 404 in each cell. The 3D matrix is generated by computing vectors for the matrix cells of the 2D matrix depicted in figure 3, thereby transforming the 2D matrix 202 into a 3D matrix 400.

Each vector is computed by comparing the genomic feature value (e.g. SNP bases A, C, G, or T) contained in a particular cell with the corresponding genomic feature values of (max. all) other sources of genetic information examined at the same genomic position represented by the matrix cell for which the vector is computed. Each genomic feature value comparison outcome can be either "identical" or "not identical". The results of these comparisons will be encoded in a vector, e.g. a vector of digits "1" or "-1 ". For example, "1" can encode for "identical" and -1 for "not identical" for instance (the encoding can also be by 1 and 0 or differently). By doing this, each genomic feature value of a matrix cell is used as a basis for computing a respective vector of digits 1, 1, 1, -1, -1 which encodes the set of comparison results for a genomic feature value within the given set of sources of genetic information.

The maximum vector length cannot be longer than the number of individuals in the population and the vector element positions always represent the same one of the sources of genetic information. The vector construction will be performed for each source of genetic information examined (as indicated by the units in the second dimension 302) and for all available genomic positions examined (as indicated by the units in the first dimension 304). Hereby all equivalent genomic feature values will instantly end up with identical vectors, because the outcome of comparison procedure within the given population will give the same results.

For example, a vector 404 is computed for a matrix cell (SGI1, GP6) indicating that organism SGI1 comprises the genomic feature "A" at genomic position GP6. The vector of this cell is computed to have the identity indicator values of 1|1|-1|-1|-1|-1| for the vector elements VE1-VE6 because:
VE1 (1): Comparing observed nucleotide "A" at GP6 of SGI1 with observed nucleotide "A" at GP6 of **SGI1** → IDENTITY
VE2 (1): Comparing observed nucleotide "A" at GP6 of SGI1 with observed nucleotide "A" at GP6 of **SGI2** → IDENTITY
VE3 (-1): Comparing observed nucleotide "A" at GP6 of SGI1 with observed nucleotide "G" at GP6 of **SGI3** → NON-IDENTITY
VE4 (-1): Comparing observed nucleotide "A" at GP6 of SGI1 with observed nucleotide "T" at GP6 of **SGI4** → NON-IDENTITY
VE5 (-1): Comparing observed nucleotide "A" at GP6 of **SGI1** with observed nucleotide "T" at GP6 of **SGI5** → NON-IDENTITY
VE6 (-1): Comparing observed nucleotide "A" at GP6 of SGI1 with observed nucleotide "T" at GP6 of **SGI6** → NON-IDENTITY

Analogously, another vector is computed for a matrix cell 306 (SGI6, GP1) indicating that organism SGI6 comprises the genomic feature "T" at genomic position GP1. The vector of cell 306 is computed to have the identity indicator values of -1|-1|-1|1|1|1| for the vector elements VE1-VE6 because:
VE1 (-1): Comparing observed nucleotide "T" at GP1 of SGI6 with observed nucleotide "A" at GP1 of **SGI1** → NON-IDENTITY
VE2 (-1): Comparing observed nucleotide "T" at GP1 of SGI6 with observed nucleotide "A" at GP1 of **SGI2** → NON-IDENTITY
VE3 (-1): Comparing observed nucleotide "T" at GP1 of SGI6 with observed nucleotide "A" at GP1 of **SGI3** → NON-IDENTITY
VE4 (1): Comparing observed nucleotide "T" at GP1 of SGI6 with observed nucleotide "T" at GP1 of **SGI4** → IDENTITY
VE5 (1): Comparing observed nucleotide "T" at GP1 of SGI6 with observed nucleotide "T" at GP1 of **SGI5** → IDENTITY
VE6 (1): Comparing observed nucleotide "T" at GP1 of SGI6 with observed nucleotide "T" at GP1 of **SGI6** → IDENTITY

As can be inferred from the above two exemplary vector computations, the genomic feature value contained in the cell for which a vector is computed is compared with the genomic feature values observed in all the sources of genetic information examined at the particular genomic position represented by the cell for which the vector is computed, whereby the comparison always is performed in a constant and predefined order of these sources to ensure that each vector element position always corresponds to the same one of the sources of genetic information. In the example depicted in figure 4, the first vector element position represents SGI1, the second vector element position represents SGI2, and so on.

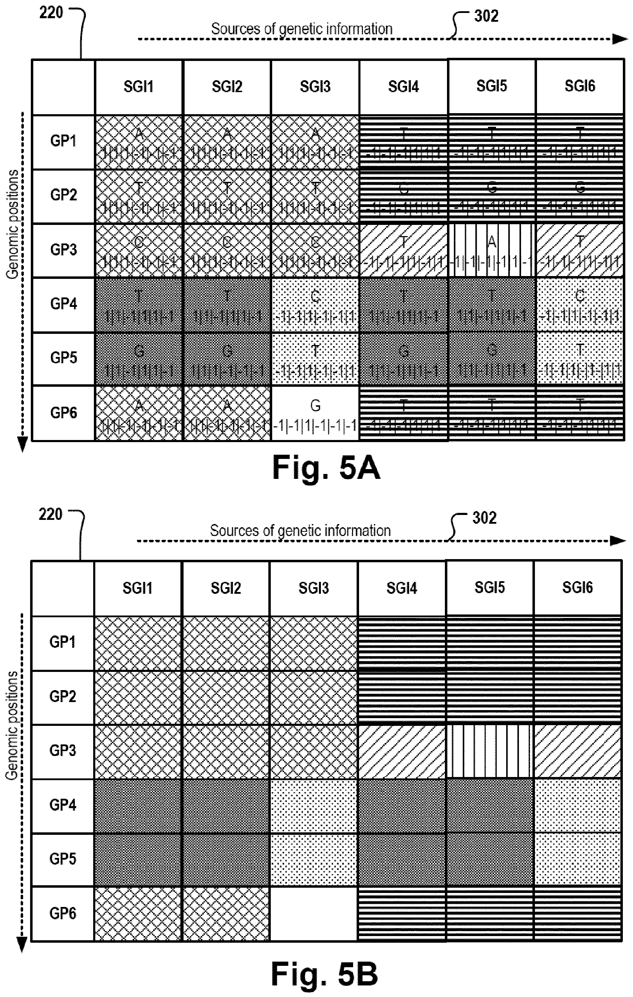

**Figure 5** depicts two versions of a haploblock plot respectively being a graphical representation of the result of a vector comparison and vector-based identification of haploblocks.

According to one embodiment (that corresponds to a very strict mode of determining similar vectors), all cells in the 3D matrix 400 having assigned the same vector are grouped together into continuous or discontinuous blocks of cells having the same color or hatching. These bocks of cells corresponding to a particular, unique vector, are referred to as "haploblocks". The edges of these haploblocks are drawn between positions of different vectors. Hereby the haploblocks can contain subsets of genotypes of the considered population.

In other embodiments, the strictness in building up the haploblocks is reduced by grouping matrix cells with similar (and not necessarily identical vectors) into the same haploblock. This concept leads to an overall extension of the block size. The similarity of the vectors can be determined by computing, for example, the Euclidian distance of the two vectors and determining if the distance is below a distance threshold.

The haploblocks can be graphically represented in a plot referred to as "haploblock plot". Thereby, population wide and genome wide equalized genomic feature values will be visually grouped together into coinherited haploblocks.

**Figure 5A** shows a haploblock plot in the form of a graphical representation of the 3D matrix 400 of figure 4, whereby matrix cell blocks sharing the same vector have assigned the same color (or hatching) while matrix cell blocks having a different vector have assigned different colors (or hatchings).

**Figure 5B** shows a haploblock plot in the form of a graphical representation of the 2D matrix 400 of figure 3, whereby matrix cell blocks sharing the same vector have assigned the same color (or hatching) while matrix cell blocks having a different vector have assigned different colors (or hatchings). The vector computation is necessary for generating the haploblock plot, but the graphical representation of the vectors is an optional feature. Hence, the vectors may not be shown, as is illustrated in figure 5B.

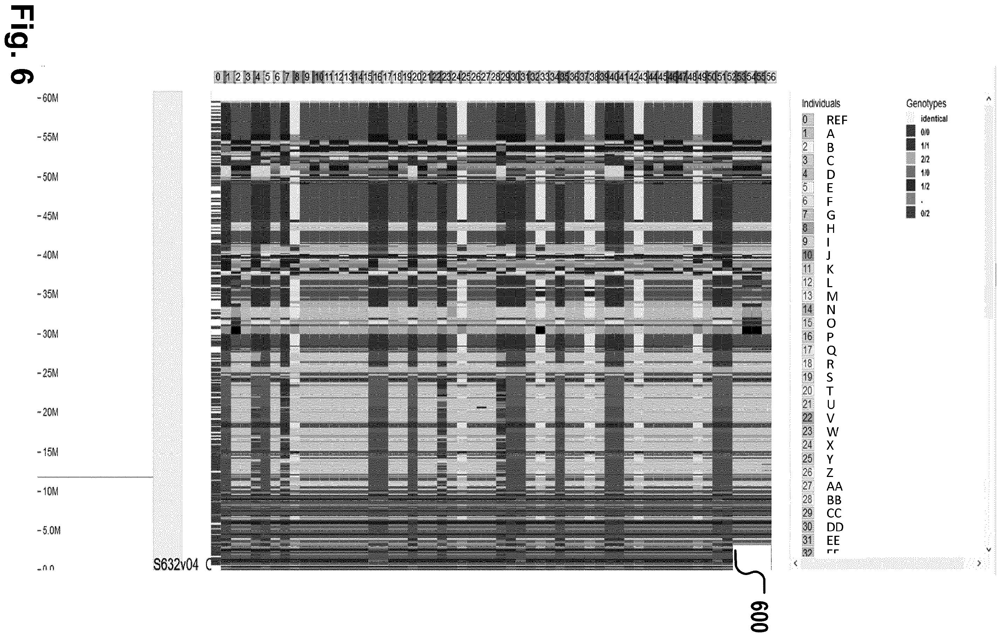

**Figure 6** is a screenshot 600 of a haploblock plot generated according to a further embodiment of the invention. In the depicted example, the "vector based haploblock identification method" is used for automatically detecting potential trait specific target regions. The screenshot shows continuous or discontinuous blocks of cells in the 2D matrix. Cells that have similar (in this embodiment: identical) vectors have the same background color. The haploblock plot shows the identified haploblocks within a population of 56 sugar beet lines (each represented in a respective column) in a genomic target region of chromosome 7. Equally colored blocks represent areas of the same vector, whereby the same vector means that all elements of the vector have, at a given vector element position, the same identity indicator value. These blocks can be considered as commonly inherited within the given set of organisms (i.e., within the given population examined). Positions of changing colors represent recombination break points and constitute cell block borders.

The screenshot 600 shows a series of blocks of the same color with different colored interruptions along the chromosome 7. All blocks of the same color consist of/represent 2D matrix cells sharing the same determined vector. Embodiments of the invention achieve a high quality of haploblock identification. Of course, the accuracy of haploblock allocation also depends on the quality of the underlying sequence data set.

According to some embodiments, the GUI comprises one or more selectable GUI elements, e.g. buttons, drop down menus, selection menus, etc. which allow a user to dynamically change the number and/or identity of one or more of the sources of genetic information covered by the second dimension of the 2D matrix. In addition, or alternatively, the GUI comprises one or more selectable GUI elements which allow a user to dynamically change the number and/or identity of the genomic positions covered by the first dimension of the 2D matrix. For example, a user can deselect and remove particular organisms or tissues used as source of genetic information and/or add sequence information of one or more additional organisms or tissue samples. The number of columns of the matrix shown in screenshot 600 and also the number of elements of all vectors 214, 404 in all matrix cells will be dynamically adapted accordingly and the haploblock plot is updated.

Hence, if the set of sources of genetic information is changed by a user, the vector-based haploblock allocation is re-computed and the set of identified haplotypes is updated in real-time. This makes the method very flexible in its application and allows an intuitive use of the haplotype identification software 210.

**Figure 7** illustrates an MSA-based version of a vector-based haplotype identification method.

**Figure 7A** illustrates the input data 704 used for the MSA and respective metadata 702 comprising positional information. The input data is provided in EMBOSS msf format and illustrates the MSA of a 50 nucleotide (nt) wide sub-sequence of a genome-wide (-several Giga-nucleotides Gnt wide) MSA performed for six organisms G1-G6 (SEQ ID NOs: 1-6).

**Figure 7B** shows a plot 706 with a type-coded version of the MSA, whereby each of the four possible DNA nucleotides A, T, G and C is represented by a respective font type (A - italic, T - bold, G - black background and italic, and C - black background and bold). The MSA is depicted in the form of 10 nt chunks. A line below the MSA is shown the consensus sequence of the alignment (SEQ ID NO: 7). The last line represents the alignment in the form of a conservation plot that allows to quickly identify mismatch positions which are represented by smaller pillars.

**Figure 7C** shows a conversion table 708 illustrating the conversion of the (10 nt) MSA chunks of figure 7B into Haplotype-sub-vectors V (also referred to as sub-vectors), whereby each vector element may comprise either the value "1" representing "identity" or "-1" representing "non-identity" to the respective 10 nt nucleotide-sub-sequence (not of individual nucleotides!) observed in other sources of genetic information G1-G6. As the MSA represents six sources G1-G6, each vector comprises six elements. The first (upper) position of each vector represents source G1, the second (second from the top) position of each vector represents G2, and so on. Applicant has observed that many MSA chunks of about 6-30 nt, in particular 10 nt, also referred to as "sub-sequences", have the same nucleotide sequence also in genetically diverse populations of organisms. This may allow reducing data size and complexity by performing a chunk-wise rather than nucleotide-wise identity check. Two sub-sequences of different sources G1, G2 have an identity indicator of "1" if their respective 10 nt DNA chunk at a particular genomic position (e.g. at A1 or A2...) are identical.

For example, all six organisms/genotypes G1-G6 have the same sub-sequence at the genomic location A1: 1-10 (SEQ ID NOs: 8-13). Hence, the vectors (or sub-sequence specific sub-vectors) obtained for each of the organisms is [1|1|1|1|1|1]. A single unique sub-vector [1|1|1|1|1|1] can be derived from all 10 nt sub-sequences observed in organisms G1-G6 at genomic position A1. This sub-vector is assigned a unique-vector-ID "H1".

The situation is different for a subsequent genomic position A2 11-20: The six organisms/genotypes G1-G6 have different sub-sequences at the genomic location A2 (SEQ ID NOs: 14-19). Hence, the vectors (or sub-sequence specific sub-vectors) obtained for each of the organisms at genomic position A2 differ from each other. From all vectors obtained for this genomic position, a unique set of vectors is automatically identified. In this case, the unique set of vectors comprises three unique vectors: [1|-1|1|-1|-1|-1] computed for G1 and G3, [-1|1|-1|1|1|-1] computed for G2, G4 and G5, and [-1|-1|-1|-1|-1|1] computed only for G6: the sequence of G6 at A2 is unique, therefore the comparison of the sub-sequence CTCTCGGATT of G6 always results in a "-1" (NON-IDENTICAL) result except for a comparison of this sub-sequence with itself (the matrix V is diagonal symmetric as both dimensions of the V matrix in figure 7C represent organisms G1-G6.

To each of these unique vectors, a unique vector-ID is assigned. For example, the vector [1|-1|1|-1|-1|-1] is assigned vector-ID H2, the vector [-1|1|-1|1|1|-1] is assigned vector-ID H3, and vector [-1|-1|-1|-1|-1|1] is assigned a vector-ID H4. A "vector-ID" is preferably a data value with a smaller size and/or lower complexity than the vector it identifies. For example, a vector-ID is preferably a single numerical or alphanumerical value. By transforming the vectors obtained for each of the sub-sequences into a vector-ID representing uniquely identified vectors for the sub-sequence in an MSA, the complexity and size can be reduced.

As can be inferred from the MSA chunks at positions A2, A4 and A5, additional unique vectors can be computed and identified, e.g. vector [-1|1|-1|1|1|1] that is assigned vector-ID H5. Vector-IDs obtained previously (e.g. H2 for position A2) may be re-used when assigning vector-IDs to vectors in subsequent genomic locations (e.g. H2 for position A3). The vector-IDs H1-H5 obtained from the MSA can be used as "genomic features" observed at a particular genomic position (here: a 10 nt sub-sequence at a particular position in the genome). Hence, the vector-IDs H1-H5 can be used as "higher-order genomic features" that can be used in a more coarse grained 2D matrix (comprising sub-vector-IDs H1-HX rather than nucleotides) that is used as a basis for computing "higher order genomic vectors", comparing the "higher-order genomic vectors" for identifying continuous or discontinuous blocks in the more coarse grained 2D matrix that respectively have similar or identical vectors and that are identified as representing a haplotype.

**Figures 7C and 7D** in combination illustrate complexity reduction: The MSA matrix for six organisms G1-G6 and for five genomic positions A1-A5 respectively comprising 10 nucleotides corresponds to a "native MSA" with 50x6 = 300 data points. After application of the vector-based haplotyping, a derivative 2D genomic feature matrix 710 is generated which comprises only 5 x 6 = 30 data points. Hence, the complexity and data size were reduced by the factor of 10.

**Figure 7E** shows a font type encoded 2D matrix 712 that is used as a haploblock plot, wherein matrix cells comprising identical sub-vector-IDs have assigned identical font type. Continuous or discontinuous blocks of cells having assigned the same font type represent a haploblock. Instead of or in addition to the use of font types, matrix cells comprised in the same haploblock can be highlighted by assigning the same type of coloring or hatching to the respective matrix cells.

**Figure 7F** shows an alternative option for graphically representing the identified haploblocks. The sequences of the respective organisms G1-G6 are graphically (by means of a font type, a color and/or hatching code) highlighted such that sub-sequences corresponding to the same vector-ID have assigned the same font type or color (as in plot 716) or hatching (as in plot 718).

As illustrated in figures 7A-7F, the vector-based haplotyping can be applied on multiple sequence alignments. The complexity reduction makes it possible to deal with very large datasets (as often the case with MSAs when large sets of organisms or tissue samples are involved). INDELS and larger PAVs can also be considered in vector- and haplotype construction. For example, missing nucleotides could result in a mismatch when the genome of an INDEL+ and an INDEL- organism are compared.

Multiple alignments often represent multiallelic states. The vector-based haplotyping method described herein is an appropriate and fast algorithm that is able to process, capture and graphically represent the respective haplotypes resulting from the multiallelic states. In fact, the vector-based haplotyping can deal with an infinite number of allelic states. The complexity reduction of the above described MSA alignment, which is based on representing vectors by unique sub-vector-IDs obtained by analyzing all sub-vectors obtained as described above for genomic sub-sequences of predefined length allows to perform whole sequence pangenome comparisons for a large number of organisms quickly and accurately. This will be of even greater impact in the future as advances in nanopore sequencing will make reference genome sequence generation cheaper by magnitude. Pangenome comparisons are very large (multi)genome wide MSAs. Vector-based haplotyping may be used to reduce this huge information to the essentials used for breeding and research: tracing inheritance in full sequenced large populations and finding causal relations between genomic variations and phenotypic traits.

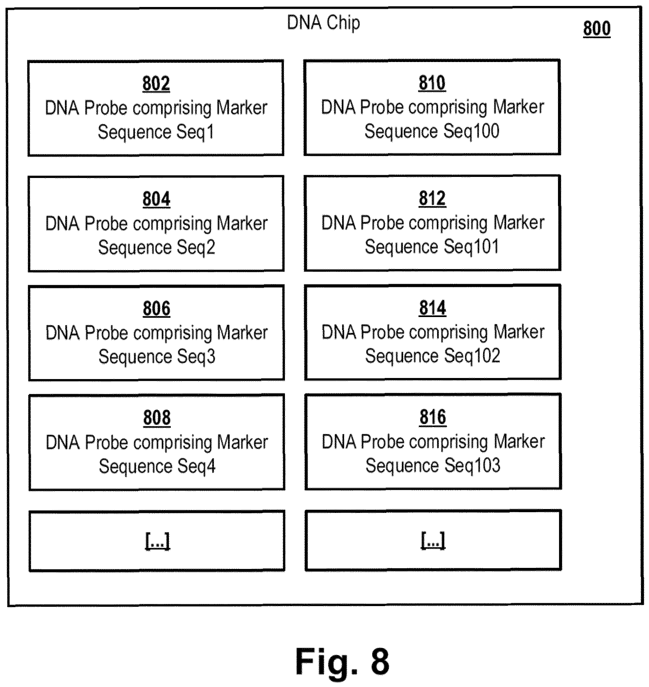

**Figure 8** is a block diagram of a DNA chip 800 also commonly known as DNA microarray. The chip comprises one or more nucleic acid probes 802 -816, e.g. DNA probes, adapted to selectively bind to nuclei acid molecules comprising one or more genetic markers being indicative of the presence of a particular gene, trait or phenotype in an organism. The genetic marker is determined by a method comprising:
- identifying consecutive or non-consecutive cell blocks using genomic information of a set of sources of genetic information by performing the computer-implemented method for identifying haplotypes according to any one of the embodiments and examples described herein and illustrated, for example, in figure 1;
- annotating the identified cell blocks; for example, the identified cell blocks can be manually or automatically be annotated with one or more genes contained in the genomic region represented by one of the blocks. In addition, or alternatively, the identified cell blocks can be manually or automatically be annotated with one or more traits or phenotypes observed in all sources of genetic information represented by the cell blocks; and
- analyzing the annotated blocks of cells for identifying one or more genetic markers associated with the presence of the particular gene, trait or phenotype.

The chip 800 can be used, for example, for selecting a germplasm comprising one or more desired genes, traits or phenotypes.

The DNA probes are arranged on the chip preferably in the form of a collection of microscopic DNA spots attached to a solid surface. Each DNA spot contains picomoles (10-12 moles) of a specific DNA sequence, known as probes (or reporters or oligos). These can be a short section of a gene or other DNA element that are used to hybridize a cDNA or cRNA (also called anti-sense RNA) sample (called target) under high-stringency conditions. Probe-target hybridization is usually detected and quantified by detection of fluorophore-, silver-, or chemiluminescence-labeled targets to determine relative abundance of nucleic acid sequences in the target.

DNA microarrays are used, according to embodiments of the invention, to genotype multiple regions of a genome, e.g. the genome of an organism that is a candidate for a breeding project.

### List of reference numerals

- 102-108: steps
- 200: computer system
- 202: main memory
- 204: processor
- 206: processor
- 208: genetic information
- 210: application program for haplotype identification
- 212: 2D matrix
- 214: vectors
- 216: identified haplotypes
- 218: display
- 220: haploblock plot
- 302: second dimension
- 304: first dimension
- 306: 2D matrix cell
- 308: 2D matrix cell
- 400: 3D matrix comprising vectors
- 404: individual vector computed for cell (SGI1, GP6)
- 600: screenshot of a further haploblock plot
- 702: meta data
- 704: input data
- 706: MSA
- 708: conversion table: MSA chunks | sub-vectors | sub-vector-IDs
- 710: 2D matrix of sub-vector IDs
- 712: haploblock plot
- 714: hatched version of plot 712
- 716: MSA with highlighted, font type-coded haploblocks
- 718: hatched version of plot 716
- 800: DNA chip
- 802-816: DNA probes

## Claims

1. A computer-implemented method for identifying haplotypes in a set of sources of genetic information, the set of sources of genetic information being a population of organisms or a set of tissues of one or more organisms, the method comprising:
- providing (102) a 2D matrix (202) comprising a first (304) and a second (302) dimension and a plurality of 2D matrix cells (306, 308),
∘ the first dimension representing a sequence of genomic positions,
∘ the second dimension representing an ordered list of the sources of genetic information,
∘ each of the plurality of cells having assigned via its respective location in the 2D matrix one of the genomic positions and one of the sources of genetic information,
∘ each of the plurality of cells comprising a genomic feature that was observed in the cell's assigned source of genetic information at the cell's assigned genomic position;
- computing (104), for each of the cells, a vector (404),
∘ the vector comprising multiple elements respectively representing one source in the set of sources of genetic information,
∘ each of the elements of the vector comprising an identity indicator, the identity indicator being a data value indicative of whether the genomic feature comprised in the cell is identical to a genomic feature observed in the source of genetic information represented by said vector element at the genomic position assigned to the cell;
- comparing (106) the vectors with each other for identifying two or more continuous or discontinuous blocks of cells in the 2D matrix that have similar vectors, wherein the vector similarity is computed as a function of the number of different alleles shared by the two compared vectors; and
outputting (108) the identified blocks of cells, each identified block of cells representing a haplotype observed in the set of sources of genetic information.

2. The computer-implemented method of claim 1, the identification of the two or more continuous or discontinuous blocks of cells in the 2D matrix that have similar vectors comprises computing the Euclidian distance between any two of the computed vectors and determining all cells whose vectors have a Euclidian distance below a predefined distance threshold value to be a member of a continuous or discontinuous block of cells having similar vectors.

3. The computer-implemented method of anyone of claim 1, the identification of the two or more continuous or discontinuous blocks of cells comprising identifying two or more continuous or discontinuous blocks of cells in the 2D matrix that have identical vectors and selectively using these identified blocks of cells as the block of cells having similar vectors.

4. The computer-implemented method of anyone of the previous claims,
- wherein the vectors are computed in parallel by at least two different processing units (204, 206); and/or
- wherein the vectors are compared with each other in parallel by at least two different processing units (204, 206).

5. The computer-implemented method of anyone of the previous claims, wherein the genomic features are of a feature type selected from a group comprising:
- an individual nucleotide;
- an insertion/deletion variation (INDEL) of one or more nucleotides;
- a gene- or exon presence or absence variation (PAV);
- a presence or absence of a simple sequence repeat marker (SSR);
- an identifier of a nucleotide-sub-sequence of predefined length;
- an identifier of a unique nucleotide-sub-sequence observed in a multiple-sequence-alignment (MSA) of the genomes of the sources of genetic information;
- an amplified fragment length polymorphism (AFLP);
- a combination of two or more of the above-mentioned feature types.

6. The computer-implemented method of anyone of the previous claims, wherein the set of sources of genetic information comprises less than 10 sources.

7. The computer-implemented method of anyone of the previous claims, the outputting comprising:
- generating a plot (220, 600, 700) comprising a graphical representation of the 2D matrix, wherein matrix cells comprised in the same identified continuous or discontinuous block of cells have the same color or the same hatching, wherein different ones of the identified cell blocks have different colors or have different hatchings;
- displaying the plot on a graphical user interface of a display device (218).

8. The computer-implemented method of anyone of the previous claims, further comprising:
- automatically annotating at least one of the identified blocks of cells with one or more genes located in a genomic region represented by the at least one identified block of cells, or enabling a user, preferably via a GUI, for manually annotating at least one of the identified blocks of cells with the one or more genes; and/or
- automatically annotating at least one of the identified blocks of cells with one or more traits observed in the sources of genomic information represented by the at least one identified block of cells, or enabling a user, preferably via a GUI, for manually annotating at least one of the identified blocks of cells with the one or more traits, the trait being an observable property of an organism, a tissue, a cell or a cell component; and/or
- automatically annotating at least one of the identified blocks of cells with one or more phenotypes observed in the sources of genomic information represented by the at least one identified block of cells, or enabling a user, preferably via a GUI, for manually annotating at least one of the identified blocks of cells with the one or more phenotypes, each phenotype being a composition of two or more traits; and
- optionally automatically analyzing the identified blocks of cells and their annotated genes for automatically identifying co-inherited genes and associated pathways, or displaying the identified cell blocks in association with their annotated genes via a GUI for enabling a user identifying co-inherited genes and associated pathways.

9. The computer-implemented method of anyone of the previous claims, further comprising:
- identifying, for each of the identified haplotypes, a predefined minimum number of genetic markers being selectively indicative of the presence of said haplotype, the predefined minimum number being independent of the length of the genomic sequence covered by the haplotype;
- selectively using the identified markers for performing an association study in a plurality of further sources of genetic information, the association study determining the co-occurrence of the identified genetic markers in the genomes of the other sources on the one hand and of genes, traits or phenotypes observed in the other sources on the other hand.

10. A method of identifying one or more genetic markers respectively associated with a gene, trait or phenotype, the method comprising:
- performing the computer-implemented method according to claim 9 for obtaining haplotypes annotated with genes, traits and/or phenotypes, whereby the set of sources of genetic information is a population of organisms;
- determining, for at least some of the identified haplotypes, one or more candidate genetic markers in the genomic region represented by said haplotype;
- analyzing correlated occurrences of the annotated haplotypes and the determined candidate genetic markers for identifying one or more candidate genetic markers observed to be associated with one or more genes, traits or phenotypes; and
- using the determined candidate genetic markers as the identified genetic markers.

11. A method of identifying a germplasm whose genome is associated with a desired first gene, trait or phenotype, the method comprising:
- performing the computer-implemented method according to claim 10 for identifying one or more first genetic markers associated with the first desired gene, trait or phenotype in the genomes of organisms of a particular species, whereby the sources of genetic information are organisms of this species;
- providing a set of germplasms of this species;
- identifying one or more first ones of the germplasms whose genome comprises the identified first genetic markers.

12. The method of claim 11, further comprising identifying second ones of the provided germplasms having a genome associated with a desired second gene, trait or phenotype, the method comprising:
- performing the computer-implemented method according to claim 10 for identifying one or more second genetic markers associated with the second desired gene, trait or phenotype in the genomes of individuals of the particular species, whereby the sources of genetic information are organisms of this species;
- identifying one or more second ones of the germplasms whose genome comprises the identified second genetic markers.

13. A method for selecting individuals of a population of organisms in a breeding program, the method comprising the steps of:
- growing a genetically diverse population of training organisms;
- phenotyping the genetically diverse population of training organisms to generate a phenotype training data set, the phenotype training data set being indicative of phenotypes and traits of the training organisms;
- identifying consecutive or non-consecutive cell blocks representing training haplotypes, the training haplotypes being haplotypes of the training organisms, by performing the computer-implemented method according to any one of claims 1-9, thereby using the genetically diverse population of training organisms as the set of sources of genetic information;
- obtaining an association training data set by associating the phenotype training data set with the training haplotypes, the association training data set being indicative of associations of some of the training haplotypes and some of the phenotypes or traits;
- identifying consecutive or non-consecutive cell blocks representing breeding haplotypes of a genetically diverse population of breeding organisms, the breeding haplotypes being haplotypes of the breeding organisms, by performing the computer-implemented method according to any one of claims 1-9, thereby using the genetically diverse population of breeding organisms as the set of sources of genetic information;
- applying the association training data set on the identified breeding haplotypes for selecting breeding pairs likely to generate progeny with one or more desired genes, traits or phenotypes.

14. A computer-readable, non-volatile storage medium (222) comprising instructions (210) which, when executed by a processor (204, 206), cause the processor to perform a method according to any one of claims 1-9.

15. A computer system comprising:
- a storage medium (222, 202) comprising a 2D matrix (202), the 2D matrix comprising first (304) and a second (302) dimension and a plurality of 2D matrix cells (306, 308),
∘ the first dimension representing a sequence of genomic positions,
∘ the second dimension representing an ordered list of sources of genetic information, the sources of genetic information being a population of organisms or a set of tissues of one or more organisms,
∘ each of the plurality of cells having assigned via its respective location in the 2D matrix one of the genomic positions and one of the sources of genetic information,
∘ each of the plurality of cells comprising a genomic feature that was observed in the cell's assigned source of genetic information at the cell's assigned genomic position;
- one or more processors (204, 206) configured for:
∘ computing (104), for each of the cells, a vector (404),
▪ the vector comprising multiple elements respectively representing one of the sources of genetic information,
▪ each of the elements of the vector comprising an identity indicator, the identity indicator being a data value indicative of whether the genomic feature comprised in the cell is identical to a genomic feature observed in the source of genetic information represented by said vector element at the genomic position assigned to the cell;
∘ comparing (106) the vectors with each other for identifying two or more continuous or discontinuous blocks of cells in the 2D matrix that have similar vectors, wherein the vector similarity is computed as a function of the number of different alleles shared by the two compared vectors; and
∘ outputting (108) the identified blocks of cells, each identified block of cells representing a haplotype observed in the sources of genetic information.

## Patentansprüche

1. Computerimplementiertes Verfahren zur Identifizierung von Haplotypen in einem Satz von Quellen genetischer Information, wobei der Satz von Quellen genetischer Information eine Population von Organismen oder ein Satz von Geweben eines oder mehrerer Organismen ist, wobei das Verfahren umfasst:
- Bereitstellen (102) einer 2D-Matrix (202), die eine erste (304) und eine zweite (302) Dimension und eine Mehrzahl von 2D-Matrixzellen (306, 308) umfasst, wobei
∘ die erste Dimension eine Sequenz von genomischen Positionen darstellt,
∘ die zweite Dimension eine geordnete Liste der Quellen genetischer Information darstellt,
∘ jeder der Mehrzahl von Zellen über ihre jeweilige Position in der 2D-Matrix eine der genomischen Positionen und eine der Quellen genetischer Information zugewiesen ist,
∘ jede der Mehrzahl von Zellen ein genomisches Merkmal umfasst, das in der der Zelle zugewiesenen Quelle genetischer Information an der der Zelle zugewiesenen genomischen Position beobachtet wurde;
- Berechnen (104) eines Vektors (404) für jede der Zellen, wobei
∘ der Vektor mehrere Elemente umfasst, die jeweils eine Quelle in dem Satz von Quellen genetischer Information darstellen,
∘ jedes der Elemente des Vektors einen Identitätsindikator umfasst, wobei der Identitätsindikator ein Datenwert ist, der angibt, ob das in der Zelle enthaltene genomische Merkmal mit einem genomischen Merkmal identisch ist, das in der Quelle genetischer Information beobachtet wird, die durch das Vektorelement an der der Zelle zugewiesenen genomischen Position repräsentiert wird,
- Vergleichen (106) der Vektoren miteinander, um zwei oder mehr kontinuierliche oder diskontinuierliche Zellblöcke in der 2D-Matrix zu identifizieren, die ähnliche Vektoren aufweisen, und
- Ausgeben (108) der identifizierten Zellblöcke, wobei jeder identifizierte Zellblock einen Haplotyp darstellt, der in dem Satz von Quellen genetischer Information beobachtet wurde.

2. Computerimplementiertes Verfahren nach Anspruch 1, wobei die Identifizierung der zwei oder mehr kontinuierlichen oder diskontinuierlichen Zellblöcke in der 2D-Matrix, die ähnliche Vektoren aufweisen, das Berechnen des euklidischen Abstands zwischen zwei beliebigen der berechneten Vektoren und das Bestimmen aller Zellen, deren Vektoren einen euklidischen Abstand unter einem vordefinierten Abstandsschwellenwert aufweisen, als Mitglied eines kontinuierlichen oder diskontinuierlichen Zellblocks mit ähnlichen Vektoren umfasst.

3. Computerimplementiertes Verfahren nach einem der Ansprüche 1, wobei die Identifizierung der zwei oder mehr kontinuierlichen oder diskontinuierlichen Zellblöcke das Identifizieren von zwei oder mehr kontinuierlichen oder diskontinuierlichen Zellblöcken in der 2D-Matrix umfasst, die identische Vektoren aufweisen, und das selektive Verwenden dieser identifizierten Zellblöcke als der Zellblock mit ähnlichen Vektoren.

4. Computerimplementiertes Verfahren nach einem der vorhergehenden Ansprüche,
- wobei die Vektoren parallel von mindestens zwei verschiedenen Verarbeitungseinheiten (204, 206) berechnet werden, und/oder
- wobei die Vektoren von mindestens zwei verschiedenen Verarbeitungseinheiten (204, 206) parallel miteinander verglichen werden.

5. Computerimplementiertes Verfahren nach einem der vorhergehenden Ansprüche, wobei die genomischen Merkmale von einem Merkmalstyp sind, der aus einer Gruppe ausgewählt ist, die umfasst:
- ein einzelnes Nukleotid;
- eine Insertions-/Deletionsvariation (INDEL) von einem oder mehreren Nukleotiden;
- eine Gen- oder Exon-Anwesenheits- oder Abwesenheitsvariation (PAV);
- eine Anwesenheit oder Abwesenheit eines einfachen Sequenzwiederholungsmarkers (SSR);
- einen Identifikator für eine Nukleotid-Teilsequenz mit vordefinierter Länge;
- einen Identifikator für eine eindeutige Nukleotid-Teilsequenz, die in einem Multiple-Sequence-Alignment (MSA) der Genome der Quellen genetischer Information beobachtet wurde;
- einen amplifizierten Fragmentlängen-Polymorphismus (AFLP);
- eine Kombination aus zwei oder mehr der oben genannten Merkmalstypen.

6. Computerimplementiertes Verfahren nach einem der vorhergehenden Ansprüche, wobei der Satz von Quellen genetischer Information weniger als 10 Quellen umfasst.

7. Computerimplementiertes Verfahren nach einem der vorhergehenden Ansprüche, wobei das Ausgeben umfasst:
- Erzeugen eines Plots (220, 600, 700), der eine grafische Darstellung der 2D-Matrix umfasst, wobei Matrixzellen, die in demselben identifizierten kontinuierlichen oder diskontinuierlichen Zellblock enthalten sind, dieselbe Farbe oder dieselbe Schraffur aufweisen, wobei verschiedene der identifizierten Zellblöcke verschiedene Farben oder verschiedene Schraffuren aufweisen,
- Anzeigen des Plots auf einer grafischen Benutzeroberfläche einer Anzeigevorrichtung (218).

8. Computerimplementiertes Verfahren nach einem der vorhergehenden Ansprüche, ferner umfassend:
- automatisches Annotieren mindestens eines der identifizierten Zellblöcke mit einem oder mehreren Genen, die sich in einer genomischen Region befinden, die durch den mindestens einen identifizierten Zellblock repräsentiert wird, oder Ermöglichen, dass ein Benutzer, vorzugsweise über eine GUI, mindestens einen der identifizierten Zellblöcke manuell mit dem einen oder mehreren Genen annotiert, und/oder
- automatisches Annotieren mindestens eines der identifizierten Zellblöcke mit einer oder mehreren Eigenschaften, die in den Quellen genomischer Information, die durch den mindestens einen identifizierten Zellblock repräsentiert wird, beobachtet werden, oder Ermöglichen, dass ein Benutzer, vorzugsweise über eine GUI, mindestens einen der identifizierten Zellblöcke manuell mit der einen oder den mehreren Eigenschaften annotiert, wobei die Eigenschaft eine beobachtbare Charakteristik eines Organismus, eines Gewebes, einer Zelle oder einer Zellkomponente ist; und/oder
- automatisches Annotieren mindestens eines der identifizierten Zellblöcke mit einem oder mehreren Phänotypen, die in den Quellen genomischer Information, die durch den mindestens einen identifizierten Zellblock repräsentiert werden, beobachtet werden, oder Ermöglichen, dass ein Benutzer, vorzugsweise über eine GUI, mindestens einen der identifizierten Zellblöcke manuell mit dem einen oder mehreren Phänotypen annotiert, wobei jeder Phänotyp eine Zusammensetzung aus zwei oder mehreren Eigenschaften ist; und
- wahlweise automatisches Analysieren der identifizierten Zellblöcke und ihrer annotierten Gene, um automatisch ko-vererbte Gene und zugeordnete Pfade zu identifizieren, oder Anzeigen der identifizierten Zellblöcke in Verbindung mit ihren annotierten Genen über eine GUI, um einem Benutzer die Identifizierung von ko-vererbten Genen und zugeordneten Pfaden zu ermöglichen.

9. Computerimplementiertes Verfahren nach einem der vorhergehenden Ansprüche, ferner umfassend:
- Identifizieren, für jeden der identifizierten Haplotypen, einer vordefinierten Mindestanzahl von genetischen Markern, die selektiv auf die Anwesenheit des Haplotyps hinweisen, wobei die vordefinierte Mindestanzahl unabhängig von der Länge der genomischen Sequenz ist, die durch den Haplotyp abgedeckt wird,
- selektives Verwenden der identifizierten Marker zur Durchführung einer Assoziationsstudie in einer Mehrzahl weiterer Quellen genetischer Information, wobei die Assoziationsstudie das gemeinsame Auftreten der identifizierten genetischen Marker in den Genomen der anderen Quellen einerseits und von Genen, Eigenschaften oder Phänotypen, die in den anderen Quellen beobachtet werden, andererseits bestimmt.

10. Verfahren zur Identifizierung eines oder mehrerer genetischer Marker, die jeweils einem Gen, einer Eigenschaft oder einem Phänotyp zugeordnet sind, wobei das Verfahren umfasst:
- Durchführen des computerimplementierten Verfahrens nach Anspruch 9 zum Erhalten von Haplotypen, die mit Genen, Eigenschaften und/oder Phänotypen annotiert sind, wobei der Satz von Quellen genetischer Information eine Population von Organismen ist;
- Bestimmen, für mindestens einige der identifizierten Haplotypen, eines oder mehrerer potenzieller genetischer Marker in der genomischen Region, die durch diesen Haplotyp repräsentiert wird,
- Analysieren des korrelierten Auftretens der annotierten Haplotypen und der bestimmten potenziellen genetischen Marker zum Identifizieren eines oder mehrerer potenzieller genetischer Marker, von denen beobachtet wurde, dass sie einem oder mehreren Genen, Eigenschaften oder Phänotypen zugeordnet sind; und
- Verwenden der bestimmten potenziellen genetischen Marker als identifizierte genetische Marker.

11. Verfahren zur Identifizierung eines Keimplasmas, dessen Genom einem/einer gewünschten ersten Gen, Eigenschaft oder Phänotyp zugeordnet ist, wobei das Verfahren umfasst:
- Durchführen des computerimplementierten Verfahrens nach Anspruch 10 zum Identifizieren eines oder mehrerer erster genetischer Marker, die dem/der ersten gewünschten Gen, Eigenschaft oder Phänotyp in den Genomen von Organismen einer besonderen Spezies zugeordnet sind, wobei die Quellen genetischer Information Organismen dieser Spezies sind;
- Bereitstellen eines Satzes von Keimplasmen dieser Spezies;
- Identifizieren eines oder mehrerer erster Keimplasmen, deren Genom die identifizierten ersten genetischen Marker umfasst.

12. Verfahren nach Anspruch 11, das ferner das Identifizieren von zweiten der bereitgestellten Keimplasmen mit einem Genom umfasst, das einem/einer gewünschten zweiten Gen, Eigenschaft oder Phänotyp zugeordnet ist, wobei das Verfahren umfasst:
- Durchführen des computerimplementierten Verfahrens nach Anspruch 10 zum Identifizieren eines oder mehrerer zweiter genetischer Marker, die dem/der zweiten gewünschten Gen, Eigenschaft oder Phänotyp in den Genomen von Individuen der besonderen Spezies zugeordnet sind, wobei die Quellen genetischer Information Organismen dieser Spezies sind;
- Identifizieren eines oder mehrerer zweiter Keimplasmen, deren Genom die identifizierten zweiten genetischen Marker umfasst.

13. Verfahren zur Auswahl von Individuen einer Population von Organismen in einem Zuchtprogramm, wobei das Verfahren die folgenden Schritte umfasst:
- Züchten einer genetisch vielfältigen Population von Trainingsorganismen;
- Phänotypisieren der genetisch vielfältigen Population von Trainingsorganismen, um einen Phänotyp-Trainingsdatensatz zu erzeugen, wobei der Phänotyp-Trainingsdatensatz auf Phänotypen und Eigenschaften der Trainingsorganismen hinweist;
- Identifizieren von konsekutiven oder nicht-konsekutiven Zellblöcken, die Trainingshaplotypen darstellen, wobei die Trainingshaplotypen Haplotypen der Trainingsorganismen sind, durch Ausführen des computerimplementierten Verfahrens nach einem der Ansprüche 1-9, wodurch die genetisch vielfältige Population von Trainingsorganismen als der Satz von Quellen genetischer Information verwendet wird;
- Erhalten eines Assoziations-Trainingsdatensatzes durch Assoziieren des Phänotyp-Trainingsdatensatzes mit den Trainings-Haplotypen, wobei der Assoziations-Trainingsdatensatz auf Assoziationen von einigen der Trainings-Haplotypen und einigen der Phänotypen oder Eigenschaften hinweist;
- Identifizieren von konsekutiven oder nicht-konsekutiven Zellblöcken, die Zuchthaplotypen einer genetisch vielfältigen Population von Zuchtorganismen darstellen, wobei die Zuchthaplotypen Haplotypen der Zuchtorganismen sind, durch Ausführen des computerimplementierten Verfahrens nach einem der Ansprüche 1-9, wodurch die genetisch vielfältige Population von Zuchtorganismen als der Satz von Quellen genetischer Information verwendet wird;
- Anwenden des Assoziations-Trainingsdatensatzes auf die identifizierten Zuchthaplotypen zur Auswahl von Zuchtpaaren, die wahrscheinlich Nachkommen mit einem/einer oder mehreren gewünschten Genen, Eigenschaften oder Phänotypen hervorbringen werden.

14. Computerlesbares, nichtflüchtiges Speichermedium (222), das Anweisungen (210) umfasst, die, wenn sie von einem Prozessor (204, 206) ausgeführt werden, den Prozessor veranlassen, ein Verfahren nach einem der Ansprüche 1-9 durchzuführen.

15. Computersystem, umfassend:
- ein Speichermedium (222, 202), das eine 2D-Matrix (202) umfasst, wobei die 2D-Matrix eine erste (304) und eine zweite (302) Dimension und eine Mehrzahl von 2D-Matrixzellen (306, 308) umfasst, wobei
∘ die erste Dimension eine Sequenz von genomischen Positionen darstellt,
∘ die zweite Dimension eine geordnete Liste von Quellen genetischer Information darstellt, wobei die Quellen genetischer Information eine Population von Organismen oder ein Satz von Geweben eines oder mehrerer Organismen sind,
∘ jeder der Mehrzahl von Zellen über ihre jeweilige Position in der 2D-Matrix eine der genomischen Positionen und eine der Quellen genetischer Information zugewiesen ist,
∘ jede der Mehrzahl von Zellen ein genomisches Merkmal umfasst, das in der der Zelle zugewiesenen Quelle genetischer Information an der der Zelle zugewiesenen genomischen Position beobachtet wurde;
- einen oder mehrere Prozessoren (204, 206), die ausgestaltet sind zum:
∘ Berechnen (104) eines Vektors (404) für jede der Zellen, wobei
▪ der Vektor mehrere Elemente umfasst, die jeweils eine der Quellen genetischer Information darstellen,
▪ jedes der Elemente des Vektors einen Identitätsindikator umfasst, wobei der Identitätsindikator ein Datenwert ist, der angibt, ob das in der Zelle enthaltene genomische Merkmal mit einem genomischen Merkmal identisch ist, das in der Quelle genetischer Information beobachtet wird, die durch das Vektorelement an der der Zelle zugewiesenen genomischen Position repräsentiert wird;
∘ Vergleichen (106) der Vektoren miteinander, um zwei oder mehr kontinuierliche oder diskontinuierliche Zellblöcke in der 2D-Matrix zu identifizieren, die ähnliche Vektoren aufweisen; und
∘ Ausgeben (108) der identifizierten Zellblöcke, wobei jeder identifizierte Zellblock einen Haplotyp darstellt, der in dem Satz von Quellen genetischer Information beobachtet wurde.

## Revendications

1. Procédé mis en oeuvre par ordinateur pour identifier des haplotypes dans un ensemble de sources d'information génétique, l'ensemble de sources d'information génétique étant une population d'organismes ou un ensemble de tissus d'un ou de plusieurs organismes, le procédé comprenant les étapes consistant à :
- fournir (102) une matrice 2D (202) comprenant une première (304) et une seconde (302) dimension et une pluralité de cellules de matrice 2D (306, 308),
∘ la première dimension représentant une séquence de positions génomiques,
∘ la seconde dimension représentant une liste ordonnée des sources d'information génétique,
∘ chacune parmi la pluralité de cellules se voyant attribuer par le biais de son emplacement respectif dans la matrice 2D l'une des positions génomiques et l'une des sources d'information génétique,
∘ chacune parmi la pluralité de cellules comprenant une caractéristique génomique qui a été observée dans la source d'information génétique attribuée à la cellule au niveau de la position génomique attribuée à la cellule ;
- calculer (104), pour chacune des cellules, un vecteur (404),
∘ le vecteur comprenant plusieurs éléments représentant respectivement une source dans l'ensemble de sources d'information génétique,
∘ chacun des éléments du vecteur comprenant un indicateur d'identité, l'indicateur d'identité étant une valeur de données indiquant si la caractéristique génomique comprise dans la cellule est identique à une caractéristique génomique observée dans la source d'information génétique représentée par ledit élément de vecteur au niveau de la position génomique attribuée à la cellule ;
- comparer (106) les vecteurs l'un à l'autre pour identifier deux blocs de cellules continus ou discontinus ou plus dans la matrice 2D ayant des vecteurs similaires, dans lequel la similarité des vecteurs est calculée en tant que fonction du nombre d'allèles différents partagés par les deux vecteurs comparés ; et
renvoyer (108) les blocs de cellules identifiés, chaque bloc de cellules identifié représentant un haplotype observé dans l'ensemble de sources d'information génétique.

2. Procédé mis en oeuvre par ordinateur selon la revendication 1, l'identification des deux blocs de cellules continus ou discontinus ou plus dans la matrice 2D ayant des vecteurs similaires comprenant les étapes consistant à calculer la distance euclidienne entre deux quelconques des vecteurs calculés et déterminer toutes les cellules dont les vecteurs ont une distance euclidienne inférieure à une valeur de seuil de distance prédéfinie pour être un membre d'un bloc de cellules continu ou discontinu ayant des vecteurs similaires.

3. Procédé mis en oeuvre par ordinateur selon l'une quelconque de la revendication 1, l'identification des deux blocs de cellules continus ou discontinus ou plus comprenant les étapes consistant à identifier deux blocs de cellules continus ou discontinus ou plus dans la matrice 2D qui ont des vecteurs identiques et utiliser sélectivement ces blocs de cellules identifiés en tant que blocs de cellules ayant des vecteurs similaires.

4. Procédé mis en oeuvre par ordinateur selon l'une quelconque des revendications précédentes,
- dans lequel les vecteurs sont calculés en parallèle par au moins deux unités de traitement différentes (204, 206) ; et/ou
- dans lequel les vecteurs sont comparés l'un à l'autre en parallèle par au moins deux unités de traitement différentes (204, 206).

5. Procédé mis en oeuvre par ordinateur selon l'une quelconque des revendications précédentes, dans lequel les caractéristiques génomiques sont d'un type de caractéristique choisi dans un groupe comprenant :
- un nucléotide individuel ;
- une variation d'insertion/délétion (INDEL) d'un ou de plusieurs nucléotides ;
- une variation de présence ou d'absence (PAV) de gène ou d'exon ;
- une présence ou une absence d'un marqueur de répétition simple de séquence (SSR) ;
- un identifiant d'une sous-séquence nucléotidique de longueur prédéfinie ;
- un identifiant d'une sous-séquence nucléotidique unique observée dans un alignement de séquences multiples (MSA) des génomes des sources d'information génétique ;
- un polymorphisme de longueur des fragments amplifiés (AFLP) ;
- une combinaison de deux des types de caractéristique susmentionnés ou plus.

6. Procédé mis en oeuvre par ordinateur selon l'une quelconque des revendications précédentes, dans lequel l'ensemble de sources d'information génétique comprend moins de 10 sources.

7. Procédé mis en oeuvre par ordinateur selon l'une quelconque des revendications précédentes, le renvoi comprenant les étapes consistant à :
- générer un graphique (220, 600, 700) comprenant une représentation graphique de la matrice 2D, dans lequel les cellules de la matrice comprises dans le même bloc de cellules continu ou discontinu identifié ont la même couleur ou le même hachage, dans lequel des blocs différents parmi les blocs de cellules identifiés ont des couleurs différentes ou des hachages différents ;
- afficher le graphique sur une interface utilisateur graphique d'un dispositif d'affichage (218).

8. Procédé mis en oeuvre par ordinateur selon l'une quelconque des revendications précédentes, comprenant en outre les étapes consistant à : - annoter automatiquement au moins l'un des blocs de cellules identifiés avec un ou plusieurs
gènes situés dans une région génomique représentée par l'au moins un bloc de cellules identifié, ou permettre à un utilisateur, préférentiellement par le biais d'une GUI, d'annoter manuellement au moins l'un des blocs de cellules identifiés avec le ou les gènes ; et/ou
- annoter automatiquement au moins l'un des blocs de cellules identifiés avec un ou plusieurs traits observés dans les sources d'information génomique représentées par l'au moins un bloc de cellules identifié, ou permettre à un utilisateur, préférentiellement par le biais d'une GUI, d'annoter manuellement au moins l'un des blocs de cellules identifiés avec le ou les traits, le trait étant une propriété observable d'un organisme, d'un tissu, d'une cellule ou d'un constituant cellulaire ; et/ou
- annoter automatiquement au moins l'un des blocs de cellules identifiés avec un ou plusieurs phénotypes observés dans les sources d'information génomique représentées par l'au moins un bloc de cellules identifié, ou permettre à un utilisateur, préférentiellement par le biais d'une GUI, d'annoter manuellement au moins l'un des blocs de cellules identifiés avec le ou les phénotypes, chaque phénotype étant une composition de deux traits ou plus ; et
- éventuellement analyser automatiquement les blocs de cellules identifiés et leurs gènes annotés pour identifier automatiquement des gènes co-hérités et les voies associées, ou afficher les blocs de cellules identifiés en association avec leurs gènes annotés par le biais d'une GUI pour permettre à un utilisateur d'identifier des gènes co-hérités et les voies associées.

9. Procédé mis en oeuvre par ordinateur selon l'une quelconque des revendications précédentes, comprenant en outre les étapes consistant à :
- identifier, pour chacun des haplotypes identifiés, un nombre minimal prédéfini de marqueurs génétiques indiquant sélectivement la présence dudit haplotype, le nombre minimal prédéfini étant indépendant de la longueur de la séquence génomique couverte par l'haplotype ;
- utiliser sélectivement les marqueurs identifiés pour réaliser une étude d'association dans une pluralité de sources d'information génétique supplémentaires, l'étude d'association déterminant la co-occurrence des marqueurs génétiques identifiés dans les génomes des autres sources d'une part et des gènes, traits ou phénotypes observés dans les autres sources d'autre part.

10. Procédé d'identification d'un ou de plusieurs marqueurs génétiques respectivement associés à un gène, un trait ou un phénotype, le procédé comprenant les étapes consistant à :
- réaliser le procédé mis en oeuvre par ordinateur selon la revendication 9 pour obtenir des haplotypes annotés avec des gènes, traits et/ou phénotypes, moyennant quoi l'ensemble de sources d'information génétique est une population d'organismes ;
- déterminer, pour au moins certains des haplotypes identifiés, un ou plusieurs marqueurs génétiques candidats dans la région génomique représentée par ledit haplotype ;
- analyser les occurrences corrélées des haplotypes annotés et des marqueurs génétiques candidats déterminés pour identifier un ou plusieurs marqueurs génétiques candidats observés à associer à un ou plusieurs gènes, traits ou phénotypes ; et
- utiliser les marqueurs génétiques candidats déterminés en tant que marqueurs génétiques identifiés.

11. Procédé d'identification d'un plasma germinatif dont le génome est associé avec un premier gène, trait ou phénotype souhaité, le procédé comprenant les étapes consistant à :
- réaliser le procédé mis en oeuvre par ordinateur selon la revendication 10 pour identifier un ou plusieurs premiers marqueurs génétiques associés au premier gène, trait ou phénotype souhaité dans les génomes d'organismes d'une espèce particulière, moyennant quoi les sources d'information génétique sont des organismes de cette espèce ;
- fournir un ensemble de plasmas germinatifs de cette espèce ;
- identifier un ou plusieurs premiers parmi les plasmas germinatifs dont le génome comprend les premiers marqueurs génétiques identifiés.

12. Procédé selon la revendication 11, comprenant en outre l'étape consistant à identifier des seconds parmi les plasmas germinatifs fournis ayant un génome associé à un second gène, trait ou phénotype souhaité, le procédé comprenant les étapes consistant à :
- réaliser le procédé mis en oeuvre par ordinateur selon la revendication 10 pour identifier un ou plusieurs seconds marqueurs génétiques associés au second gène, trait ou phénotype souhaité dans les génomes d'individus de l'espèce particulière, moyennant quoi les sources d'information génétique sont des organismes de cette espèce ;
- identifier un ou plusieurs seconds parmi les plasmas germinatifs dont le génome comprend les seconds marqueurs génétiques identifiés.

13. Procédé de sélection d'individus d'une population d'organismes dans un programme de reproduction, le procédé comprenant les étapes consistant à :
- faire croître une population génétiquement diverse d'organismes d'entraînement ;
- phénotyper la population génétiquement diverse d'organismes d'entraînement pour générer un ensemble de données d'entraînement de phénotype, l'ensemble de données d'entraînement de phénotype indiquant des phénotypes et des traits des organismes d'entraînement ;
- identifier des blocs de cellules consécutifs ou non consécutifs représentant des haplotypes d'entraînement, les haplotypes d'entraînement étant des haplotypes des organismes d'entraînement, en réalisant le procédé mis en oeuvre par ordinateur selon l'une quelconque des revendications 1 à 9, utilisant ainsi la population génétiquement diverse d'organismes d'entraînement en tant qu'ensemble de sources d'information génétique ;
- obtenir un ensemble de données d'entraînement d'association en associant l'ensemble de données d'entraînement de phénotype avec les haplotypes d'entraînement, l'ensemble de données d'entraînement d'association indiquant des associations de certains des haplotypes d'entraînement et de certains des phénotypes ou traits ;
- identifier des blocs de cellules consécutifs ou non consécutifs représentant des haplotypes de reproduction d'une population génétiquement diverse d'organismes de reproduction, les haplotypes de reproduction étant des haplotypes des organismes de reproduction, en réalisant le procédé mis en oeuvre par ordinateur selon l'une quelconque des revendications 1 à 9, utilisant ainsi la population génétiquement diverse d'organismes de reproduction en tant qu'ensemble de sources d'information génétique ;
- appliquer l'ensemble de données d'entraînement d'association aux haplotypes de reproduction identifiés pour sélectionner des couples de reproduction susceptibles de générer une progéniture avec un ou plusieurs gènes, traits ou phénotypes souhaités.

14. Support de stockage non volatil lisible par ordinateur (222) comprenant des instructions (210) qui, lorsqu'elles sont exécutées par un processeur (204, 206), amènent le processeur à réaliser un procédé selon l'une quelconque des revendications 1 à 9.

15. Système informatique comprenant :
- un support de stockage (222, 202) comprenant une matrice 2D (202), la matrice 2D comprenant une première (304) et une seconde (302) dimension et une pluralité de cellules de matrice 2D (306, 308),
∘ la première dimension représentant une séquence de positions génomiques,
∘ la seconde dimension représentant une liste ordonnée de sources d'information génétique, les sources d'information génétique étant une population d'organismes ou un ensemble de tissus d'un ou de plusieurs organismes,
∘ chacune parmi la pluralité de cellules se voyant attribuer par le biais de son emplacement respectif dans la matrice 2D l'une des positions génomiques et l'une des sources d'information génétique,
∘ chacune parmi la pluralité de cellules comprenant une caractéristique génomique qui a été observée dans la source d'information génétique attribuée à la cellule au niveau de la position génomique attribuée à la cellule ;
- un ou plusieurs processeurs (204, 206) conçus pour :
∘ calculer (104), pour chacune des cellules, un vecteur (404),
▪ le vecteur comprenant plusieurs éléments représentant respectivement l'une des sources d'information génétique,
▪ chacun des éléments du vecteur comprenant un indicateur d'identité, l'indicateur d'identité étant une valeur de données indiquant si la caractéristique génomique comprise dans la cellule est identique à une caractéristique génomique observée dans la source d'information génétique représentée par ledit élément de vecteur au niveau de la position génomique attribuée à la cellule ;
∘ comparer (106) les vecteurs l'un à l'autre pour identifier deux blocs de cellules continus ou discontinus ou plus dans la matrice 2D ayant des vecteurs similaires, dans lequel la similarité des vecteurs est calculée en tant que fonction du nombre d'allèles différents partagés par les deux vecteurs comparés ; et
∘ renvoyer (108) les blocs de cellules identifiés, chaque bloc de cellules identifié représentant un haplotype observé dans les sources d'information génétique.
